(19)

Europäisches
Patentamt
European
Patent Office
Office européen
des brevets

(11) **EP 2 698 169 A1**

(12) **EUROPEAN PATENT APPLICATION**

(43) Date of publication:
**19.02.2014 Bulletin 2014/08**

(51) Int Cl.:
***A61K 49/00*** *(2006.01)*

(21) Application number: **12005859.9**

(22) Date of filing: **13.08.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Universität Duisburg-Essen
45141 Essen (DE)**

(72) Inventors:
• **Bayer, Peter**
**45147 Essen (DE)**
• **Hoffmann, Daniel**
**45133 Essen (DE)**
• **Grum, Daniel**
**44625 Herne (DE)**
• **Franke, Stefan**
**44789 Bochum (DE)**
• **Heider, Dominik**
**40215 Düsseldorf (DE)**

• **Schramm, Alexander**
**45131 Essen (DE)**
• **Müller, Jan**
**35041 Marburg (DE)**
• **Diebolder, Philipp**
**69118 Heidelberg (DE)**
• **Krauss, Jürgen**
**69259 Wilhelmsfeld (DE)**
• **Kraff, Oliver**
**45355 Essen (DE)**

(74) Representative: **Lahrtz, Fritz
Isenbruck Bösl Hörschler LLP
Patentanwälte
Prinzregentenstrasse 68
81675 München (DE)**

Remarks:
If details are not fully discernible, that is due to the
quality of the drawings filed. The meaningfulness of
the drawings lies within the applicant's responsibility.

(54) **A contrast agent-binding polypeptide and use thereof**

(57)    The present invention refers to a polypeptide comprising at least one first domain for binding to at least one contrast agent and at least one second domain for binding to at least one target structure-binding compound. Furthermore, the invention relates to said polypeptide for use in a method for the diagnosis of a patient. Moreover, the present invention embraces means and methods for producing said polypeptide and to *in vivo* and *in vitro* uses thereof.

EP 2 698 169 A1

SEQUENCE LISTING

<110> Universität Duisburg-Essen

<120> A Contrast Agent-Binding Polypeptide and Use Thereof

<130> P68120EP

<160> 9

<170> PatentIn version 3.5

<210> 1
<211> 40
<212> DNA
<213> artificial sequence

<220>
<223> forward primer for Z-domain subcloning

<400> 1
cacacagggc ccgtggataa caaatttaac aaagaacagc          40

<210> 2
<211> 36
<212> DNA
<213> artificial sequence

<220>
<223> reverse primer for Z-domain subcloning

<400> 2
ggttggggat ccattatttc ggcgcctgcg catcgt          36

<210> 3
<211> 35
<212> DNA
<213> artificial sequence

<220>
<223> forward primer for rat S55D/E59D-alpha-parvalbumin subcloning

<400> 3
cacacagggc ccagcatgac cgatctgctg agcgc          35

<210> 4
<211> 33
<212> DNA
<213> artificial sequence

<220>
<223> reverse primer for rat S55D/E59D-alpha-parvalbumin subcloning

<400> 4
ggttggggat ccattagctt tccgccacca ggg          33

<210> 5
<211> 24
<212> DNA
<213> artificial sequence

<220>
<223> forward primer for cloning a D72C mutant of Zalbin

<400> 5
ggcagcatga cctgtctgct gagc                                                    24


<210> 6
<211> 24
<212> DNA
<213> artificial sequence

<220>
<223> reverse primer for cloning a D72C mutant of zalbin

<400> 6
gctcagcaga caggtcatgc tgcc                                                     24


<210> 7
<211> 109
<212> PRT
<213> Rattus norvegicus

<400> 7

Ser Met Thr Asp Leu Leu Ser Ala Glu Asp Ile Lys Lys Ala Ile Gly
1               5                   10                  15


Ala Phe Thr Ala Ala Asp Ser Phe Asp His Lys Lys Phe Phe Gln Met
                20              25                  30


Val Gly Leu Lys Lys Lys Ser Ala Asp Asp Val Lys Lys Val Phe His
        35              40                  45


Ile Leu Asp Lys Asp Lys Asp Gly Phe Ile Asp Glu Asp Glu Leu Gly
        50              55                  60


Ser Ile Leu Lys Gly Phe Ser Ser Asp Ala Arg Asp Leu Ser Ala Lys
65                  70                  75                  80


Glu Thr Lys Thr Leu Met Ala Ala Gly Asp Lys Asp Gly Asp Gly Lys
                85                  90                  95


Ile Gly Val Glu Glu Phe Ser Thr Leu Val Ala Glu Ser
                100                 105


<210> 8
<211> 58
<212> PRT
<213> Staphylococcus aureus

<400> 8

Val Asp Asn Lys Phe Asn Lys Glu Gln Gln Asn Ala Phe Tyr Glu Ile
1               5                   10                  15


Leu His Leu Pro Asn Leu Asn Glu Glu Gln Arg Asn Ala Phe Ile Gln
                20              25                  30


Ser Leu Lys Asp Asp Pro Ser Gln Ser Ala Asn Leu Leu Ala Glu Ala
                35              40                  45

```
Lys Lys Leu Asn Asp Ala Gln Ala Pro Lys
    50                  55
```

```
<210>  9
<211>  177
<212>  PRT
<213>  Artificial Sequence

<220>
<223>  contrast agent-binding polypeptide Zalbin

<400>  9
```

```
Val Asp Asn Lys Phe Asn Lys Glu Gln Gln Asn Ala Phe Tyr Glu Ile
1               5                   10                  15
```

```
Leu His Leu Pro Asn Leu Asn Glu Glu Gln Arg Asn Ala Phe Ile Gln
            20                  25                  30
```

```
Ser Leu Lys Asp Asp Pro Ser Gln Ser Ala Asn Leu Leu Ala Glu Ala
            35                  40                  45
```

```
Lys Lys Leu Asn Asp Ala Gln Ala Pro Lys Gly Gly Gly Gly Gly Gly
    50                  55                  60
```

```
Gly Gly Gly Gly Ser Met Thr Asp Leu Leu Ser Ala Glu Asp Ile Lys
65                  70                  75                  80
```

```
Lys Ala Ile Gly Ala Phe Thr Ala Ala Asp Ser Phe Asp His Lys Lys
                85                  90                  95
```

```
Phe Phe Gln Met Val Gly Leu Lys Lys Lys Ser Ala Asp Asp Val Lys
            100                 105                 110
```

```
Lys Val Phe His Ile Leu Asp Lys Asp Lys Asp Gly Phe Ile Asp Glu
            115                 120                 125
```

```
Asp Glu Leu Gly Ser Ile Leu Lys Gly Phe Ser Ser Asp Ala Arg Asp
    130                 135                 140
```

```
Leu Ser Ala Lys Glu Thr Lys Thr Leu Met Ala Ala Gly Asp Lys Asp
145                 150                 155                 160
```

```
Gly Asp Gly Lys Ile Gly Val Glu Glu Phe Ser Thr Leu Val Ala Glu
                165                 170                 175
```

```
Ser
```

**Description**

The present invention refers to a polypeptide comprising at least one first domain for binding to at least one contrast agent and at least one second domain for binding to at least one target structure-binding compound. Furthermore, the invention relates to said polypeptide for use in a method for the diagnosis of a patient. Moreover, the present invention embraces means and methods for producing said polypeptide and to *in vivo* and *in vitro* uses thereof.

During the last decades, molecular imaging, *in vivo* as well as in *in vitro,* gained increasing importance in medicinal diagnostics and scientific research. Typically, molecular imaging is based on the visualization of the detected contrast between target structures of interest on the one hand and other areas in the patient's body or in an *in vitro* specimen on the other hand. Evidently, providing enough contrast is of crucial importance in order to enable the accurate visualization of distinct target structures. In order to improve contrast and information content, numerous contrast agents are employed. Exemplarily, for magnetic resonance imaging (MRI), one of the most widely used contrast agent is gadolinium(iii) ($Gd^{3+}$). Commercially available $Gd^{3+}$-based contrast agents are, beside others, gadopentetic acid (Magnevist®) and Dotarem®, gadodiamide (Omniscan®), gadoteridol (Prohance®), Gadovist®, gadoversetamide (OptiMARK®), gadofosveset (Vasovist®, Ablavar®), Gadocoletic acid, Gadomelitol and Gadomer 17, Primovist™, gadobenic acid (Multihance®) and gadoxetic acid (Eovist®, Primovist®).

Further, a couple of high-molecular weight compounds to which $Gd^{3+}$ is bound via synthetical chemical $Gd^{3+}$-complexing chelators such as DTPA (diethylene triamine pentaacetic acid, pentetic acid) or DOTA (1,4,7,10-tetraazacyclododecane-1,4,7,10-tetraacetic acid) covalently linked with said high-molecular weight compounds. Such high-molecular weight compounds are, exemplarily, human serum albumin (HSA), virus capsids, peptides and protein multimers. The conjugates of $Gd^{3+}$-complexing chelators and high-molecular weight compounds have been developed in order to reduce toxicity and to improve acceptance and circulation time of the contrast agent in the patient's body.

However, a severe drawback of the aforementioned compounds, independent on whether they are low- or high-molecular weight compounds, is that they bear a low relaxivity and, thus, low T1 contrast amplification (cf., e.g, Liepold et al., 2009, Nano Lett. 9(12):4520-6). Moreover, all of the aforementioned compounds are only usable to visualize vascularized structures of the patient's body such as vessels or the gastrointestinal tract and are not well suited for signal enhancing of specific and distinct structures within the patient or *in vitro* specimen.

Therefore, in the last years, it had been recognized that it is of high importance to develop contrast agent-comprising compounds bearing a high relaxivity that are able to bind specifically to distinct structures and, thereby, enable visualization of said structures inside the patient or in an *in vitro* specimen. For instance, in cancer diagnostics, it is of great interest to selectively visualize large and small tumors and metastases and cell clusters *in vivo.*

For this reason, several selectively binding $Gd^{3+}$-complexing compounds based on selectively binding small peptides and a synthetical chemical $Gd^{3+}$-complexing chelators covalently linked therewith have been developed.

These peptide-chelator complexes, however, still bear a comparably low relaxivity and insufficient specificity and are degraded rapidly when used *in vivo.* Further, the provision of these compounds is very laborious as the peptide-chelator complexes have to be synthesized by solid or liquid phase chemical peptide synthesis wherein the use of toxic reagents is indispensable and, subsequent to the synthesis, these toxic agents have to be removed from the peptide-chelator complexes prior to being administered into a patient via complicated and laborious purification steps. Moreover, each peptide-chelator complex is suitable to bind to and detect a single molecular target structure only. Therefore, for every molecular target structure of interest, a new peptide-chelator complex has to be synthesized.

In the view of the above, there is still an unmet need for a tool for molecular imaging specifically binding to a molecular target structure with high affinity and reflexivity. Desirably, this tool is further easier to produce and/or usable to specifically detect a variety of molecular target structures.

As shown in the examples of the present invention, a tool that enables specific binding to a molecular target structure with high affinity and relaxivity that is comparably easy to produce and that allows the specific detection of a variety of molecular target structures was surprisingly found.

In a first aspect, the present invention relates to a polypeptide comprising:

(a) at least one first domain for binding to at least one contrast agent; and
(b) at least one second domain for binding to at least one target structure-binding compound.

As used throughout the present invention, the term "polypeptide" may be understood interchangeably with the terms "peptide" and "protein" in the broadest sense as any molecule that is mainly composed of amino acid moieties linked by amide bonds with another and comprises at least four consecutive amino acid moieties. Herein, the terms "amide bond" and "peptidic bond" may be understood interchangeably. Likewise, also the terms "moiety", "radical" and "residue" may be understood interchangeably. Preferably, the polypeptide of the present invention comprises at least ten consecutive amino acids, at least 20 consecutive amino acids, at least 30 consecutive amino acids, at least 40 consecutive amino acids, at least 50 consecutive amino acids, at least 60 consecutive amino acids, at least 70 consecutive amino acids, at least 80 consecutive amino acids, at least 90 consecutive amino acids or at least 100 consecutive amino acids.

The polypeptide of the present invention may comprise one or more linear or branched chain(s) of consecutive amino acid moieties. Preferably the polypeptide of the present invention comprises one or more linear chain(s) of consecutive amino acid moieties. More preferably the polypeptide of the present invention consists of one linear chain of consecutive amino acid moieties. Preferably, the majority of amino acid moieties are natural amino acid moieties linked via amide bonds with another. However, the polypeptide may also consist of or comprise one or more non-natural, artificial moiety/moieties (e.g., beta amino acid moieties, D-amino acid moieties, alkylated amino acid moieties (e.g., methylated amino acid moieties), aminoxy acid moieties, hydrazine acid moieties, etc.) and/or may comprise one or more further backbone modification(s) (e.g., amide analogues instead of amide bonds (e.g., ketomethylene, vinyl, ketodifluoromethylene, amine, cyclopropene, thioester, sulfinamide, sulphonamide, phosphonamide, hydroxyethylene, E-alkene)) replacing one or more amide bond(s). Further, the peptide may also be a depsipeptide, a borapeptide, an endothiopeptide, a carbapeptide or an azapeptide.

Moreover, the amino acid backbone may be modified (e.g., by methylation, alkylation, oxidation, cyclization, dehydration). In particular the polypeptide may or may not be subjected to one or more posttranslational modification (s) and/or be conjugated with one or more non-amino acid moiety/moieties. The termini of the polypeptide may, optionally, be capped by any means known in the art, such as, e.g., amidation, acetylation, methylation, acylation. Posttranslational modifications are well-known in the art and may be but may not be limited to lipidation, phosphorylation, sulfatation, glycosylation, truncation, cyclization of several amino acid moieties, cyclization of the polypeptide strand, oxidation, reduction, decarboxylation, acetylation, amidation, deamidation, disulfide bond formation, pyroglutamate formation, amino acid addition, cofactor addition (e.g., biotinylation, heme addition, eicosanoid addition, steroid addition) and complexation of metal ions, non-metal ions, peptides or small molecules and addition of iron-sulphide clusters. Moreover, optionally, co-factors, such as the binding of ATP, ADP, $NAD^+$, $NADH+H^+$, $NADP^+$, $NADPH+H^+$, metal ions, anions, lipids, etc. may be bound to the polypeptide, irrespective on the biological influence of these co-factors.

A further moiety with which the polypeptide is optionally conjugated with may exemplarily be a labeling moiety. As used in the context of the present invention, the term "labeling moiety" may be understood in the broadest sense as any moiety that can be detected, in particular by a molecular imaging method. Exemplarily, a labeling moiety may be a fluorescence label such as, e.g., a fluorescent polypeptide (e.g., cyan fluorescent protein (CFP), green fluorescent protein (GFP) or yellow fluorescent protein (YFP), red fluorescent protein (RFP), mcherry, etc.), a small-molecule dye (e.g., an Atto dye (e.g., ATTO 390, ATTO 425, ATTO 465, ATTO 488, ATTO 495, ATTO 520, ATTO 532, ATTO 550, ATTO 565, ATTO 590, ATTO 594, ATTO 610, ATTO 611X, ATTO 620, ATTO 633, ATTO 635, ATTO 637, ATTO 647, ATTO 647N, ATTO 655, ATTO 665, ATTO 680, ATTO 700, ATTO 725, ATTO 740), a Cy dye (e.g., Cy3, Cy5, Cy5.5, Cy 7), an Alexa dye (e.g., Alexa Fluor 488, Alexa Fluor 546, Alexa Fluor 647, Alexa Fluor 680, Alexa Fluor 750), a Visen dye (e.g. VivoTag680, VivoTag750), an S dye (e.g., S0387), a DyLight fluorophore (e.g., DyLight 750, DyLight 800), an IRDye (e.g., IRDye 680, IRDye 800), a fluorescein dye (e.g., fluorescein, carboxyfluorescein, fluorescein isothiocyanate (FITC)), a rhodamine dye (e.g., rhodamine, tetramethylrhodamine (TAMRA)) or a HOECHST dye), a quantum dot or a combination of two or more thereof. A fluorescence label may optionally also be conjugated with the polypeptide to enable the easier determination of the polypeptide concentration. When the average number of fluorescent labels per polypeptide molecule and the extinction coefficient of each fluorescent label are known, the concentration may be determined by measuring the absorbance and calculating the concentration by means of the Beer-Lambert Law. Alternatively or additionally, the labeling moiety may be a spin label such as, e.g., $^2$H or $^{13}$C suitable for Nuclear Magnetic Resonance (NMR). Alternatively or additionally, the labeling moiety may be radioactive label such as, e.g., $^3$H, $^{14}$C, $^{123}$I, $^{124}$I, $^{131}$I, $^{32}$P, $^{99m}$Tc or lanthanides (e.g., $^{64}$Gd). In this context, a radioactive label may or may not be suitable for scintillation assays, computer tomography (CT), single-photon emission computed tomography (SPECT) or as a label suitable for Positron Emission Tomography (PET) (e.g., $^{11}$C, $^{13}$N, $^{15}$O, $^{18}$F, $^{82}$Rb). Alternatively or additionally, the labeling moiety may be metal atom, metal ion or metal bead (e.g., a gold bead).

As used throughout the present invention, the term "conjugated with" may be understood in the broadest sense as any kind of covalent or non-covalent attachment or linkage of one component with another component, preferably via a covalent linkage. It is not limited to a specific kind of formation of a conjugate. Depending on the components conjugated with another, such conjugate can be obtained by chemical means and/or by genetic engineering and biotechnological means.

The labelling moiety may also be a lipid or a lipidoid, a small molecule dye (e.g., a luminescent dye, an UV/Vis dye (e.g., a p-nitrophenyl moiety, Coomassie Brilliant Blue G-250)), a binding moiety (e.g., biotin, methotrexate, a glycocorticoid), an insoluble polymer (e.g., methacrylate, polystyrene (PS), polyethylene (PE), polypropylene (PP)), a soluble polymer (e.g., polyethylene glycol (PEG), hydroxypropyl methacrylate (HPMA), polyethylene imine (PEI)), an antibody (e.g., a Fab fragment, a single chain antibody, a diabody, a triabody, a flexibody, a tandab), another polypeptide, a polypeptide tag for purification purposes (e.g., a polyhistidine tag, streptavidin, dihydrofolate reductase (DHFR), a glycocorticoid receptor), an enzyme label (e.g., penicillinase, horseradish peroxidase, alkaline phosphatase), a micro- or nanobead (e.g., a functionalized silica bead, a polysaccharide-based bead), a polymersome, a micelle and/or a liposome.

Optionally, the polypeptide of the present invention may also be conjugated with one or more cell-penetrating peptide(s) (CPP(s)) (e.g., HIV Tat-1 derived CPP, penetratin (antennapedia), W/R penetratin, lactoferrin-derived CPP, transportan, chariot, HIV-gp41 fusion sequence, polyarginine ($R_n$) (e.g., heptaarginine ($R_7$) octaarginine ($R_8$), non-aarginine ($R_9$), decaarginine ($R_{10}$)), Kaposi FGF signal peptide, human integrin beta-3-signal sequence, SynB1, buforin, magainin, KLAKKLAK model peptide, loligomer, proline-rich dendrimers, N-terminal repetitive domain of maize gamma-zein, MPG, Pre2-TLM, signal transduction peptide(s) (e.g., calcitonin-derived peptide)). As used herein, the terms "cell-penetrating peptide" and "protein transduction domain" (PTD(s)) may be understood interchangeably. As well-known in the art, conjugation with one or more CPP(s) may enable the cellular import of the polypeptide of the present invention into intracellular vesicles, into the cytoplasm and/or even into the inner of the nucleus of cells and, thereby, enable the detection of an intracellular molecular target structure. Moreover, conjugation with one or more CPP(s) may enable to overcome a physiological barrier such as, e.g., the blood-brain barrier, the blood-testis barrier or the placental barrier. A CPP may be comprised in the polypeptide strand in any position such as, e.g., N-terminally from the at least one first domain and the at least one second domain, between the at least one first domain and the at least one second domain, C-terminally from the at least one first domain and the at least one second domain or may be conjugated via a side chain of the polypeptide. Such CPP may, optionally, form a part of the polypeptide strand via genetic engineering.

Optionally, the polypeptide of the present invention may also be conjugated with one or more solid support(s). As used in the context of the present invention, the term "solid support" may be understood in the broadest sense as any solid material the polypeptide may be immobilized on covalently or non-covalently. The solid support may, exemplarily, be a microarray surface (e.g., a glass surface or a hydrogel surface), a bead structure (e.g., a magnetic bead or an affinity chromatography bead), a filter, a tubing or a gel matrix.

The one or more labelling moiety/moieties, one or more CPP(s) and/or one or more solid support(s) may optionally be conjugated to the polypeptide by any chemical means known in the art (e.g., via an active ester, via an isothiocyanate, via a maleimid, via an N-hydroxysuccinimidyl ester, via a glutaraldehyde derivative, via a carbodiimide, by click chemistry, by native chemical ligation (NCL) or a combination of two or more thereof). Preferably, the one or more labelling moiety/moieties and/or CPP(s) may also be conjugated via a bivalent linker.

It will be understood that the present invention also encompasses any salts of the polypeptide described above, in particular pharmaceutically acceptable salts.

The polypeptide may optionally be dissolved in any medium suitable for storing said polypeptide such as, e.g., water, an aqueous buffer (e.g., a Hepes, Tris, or phosphate buffer), blood, serum, plasma, lymph or an organic solvent (e.g., dimethyl sulfoxide (DMSO), dimethylformide (DMF)). It will further be understood that the polypeptide thereof may be stored at conditions suitable for it, such as, e.g., in solution at room temperature, in solution in a cooled environment (e.g., at 4°C), in frozen (at -20°C), deep-frozen (e.g., -80°C, -196°C), freeze-dried and/or dry state (e.g., spray-dried) depending on its chemical nature.

As mentioned above, a polypeptide according to the present invention comprises at least two domains, i.e., at least one first domain for binding to at least one contrast agent and at least one second domain for binding to at least one target structure-binding compound.

In the context of the present invention, the term "domain" may be understood in the broadest sense as a polypeptide domain, thus, as a part of a polypeptide that may form a compact three-dimensional structure involving one or more secondary structure element(s) (e.g., one or more alpha helical structure element(s), one or more beta-folded structure element(s), one or more random coil structure element(s) and/or one or more turn structure element(s)). The tertiary structure may optionally be independently stable and folded. Typically, a polypeptide domain has a particular function such as, e.g., a binding function such as, e.g., by forming a binding site that may, exemplarily, be a binding pocket and/or a complexing structure.

As used herein, the designation of a domain as "first domain" or "second domain" should not be understood in a way that the first or second domain had a particular position in the polypeptide sequence. Rather, the position of the one or more first domain(s) and the one or more secondary domain(s), respectively, may be freely chosen. Optionally, the one or more first domain(s) may be located N-terminally from the one or more second domain(s), may be located C-terminally from the one or more second domain(s), and/or one or more first domain(s) may be located N-terminally and one or more further first domain(s) may be located C-terminally from the one or more second domain(s), and/or one or more second domain(s) may be located N-terminally and one or more further second domain(s) may be located C-terminally from the one or linker sequence comprising one or more further amino acid residue(s). As used herein the terms "linker" and "spacer" may be understood interchangeable. Preferably, the linker is a consecutive strand of natural amino acids conjugated with another via amide bonds. Even more preferably, the linker is a linker composed of between one and 100 amino acid residues, even more preferably, between five and 50 amino acid residues, even more preferably between five and 20 amino acid residues, even more preferably of between five and 15 amino acid residues, most preferably of about ten amino acid residues. Highly preferably, the linker is a polyglycine residue, in particular a polyglycine residue comprising about ten consecutive glycine residues. Alternatively, the linker sequence may also comprise one or more domain(s) that may or may not bear a particular functionality such as, e.g., a binding and/or a cell-penetrating functionality.

Highly preferably, the polypeptide is a two-domain polypeptide comprising one first domain and one second domain. Herein, preferably, the first domain and the second domain is covalently conjugated with another via a linker, wherein the linker is most preferably a liner polypeptide strand. Further, the C-terminus of the second domain (i.e., the domain binding to at least one target structure-binding compound) may herein preferably bound to the N-terminus of the first domain (i.e., the domain binding to at least one contrast agent) via a linker.

As stated above, in the context of the present invention, the first domain is suitable for binding to at least one contrast agent. The first domain may bind to the contrast agent non-covalently or covalently. Preferably, the first domain binds to the contrast agent non-covalently. Most preferably, the first domain binds to the contrast agent by complex formation. Herein, preferably, the binding site for the contrast agent is formed by two or more amino acid moieties of the polypeptide strand.

In the context of the present invention, the second domain is suitable for binding to at least one target structure-binding compound. Herein, the target structure-binding compound may be any compound known in the art that selectively binds to a molecular target structure. As used herein, the terms "selective binding" and "distinct binding" may be understood interchangeably. As used in this context, selective binding means that the ratio between the structure-binding compound binding to its molecular target structure and said compound binding to other molecular structures (off target structures) (i.e., the target structure binding : off-target structure binding ratio) is at least 5:1, at least 10:1, at least 50:1, at least 100:1, at least 1000:1 1 or at least 10000:1. As used throughout the present invention, in particular in the context of a target structure binding compound, the terms "binding" and "recognizing" may be understood interchangeably. When the polypeptide is visualizable by any means of molecular imaging, such selective binding may also be understood as staining the target structure.

As used in the context of the present invention, the molecular target structure may be any structure that is specifically recognized and bound by the structure-binding compound. The molecular target structure compound may be a high molecular weight compound of a molecular weight of 5 kDa or more, a small molecule of a molecular weight of less than 5 kDa or a haptene formed of at least one high-molecular weight compound and at least one other molecular structure. Preferably, the molecular target structure is a high molecular weight compound of a molecular weight of 5 kDa or more or a haptene.

The molecular target structure may comprise or may be at least one molecular structure that is present in a patient's body or an *in vitro* sample inherently. The molecular target structure may comprise or may be at least one molecular structure administered to a patient or an *in vitro* sample or may comprise or may be at least one haptene comprising at least one molecular structure present in a patient's body or in an *in vitro* sample inherently and at least one molecular structure administered to said patient or *in vitro* sample.

Preferably, the molecular target structure may comprise at least one molecular structure that is present in a patient's body or in an *in vitro* sample inherently. Exemplarily, the molecular target structure may comprise or be a polypeptide, a polysaccharide, a fatty acid, an eicosanoid, a steroid or a combination or two or more thereof. The molecular target structure may be located anywhere in the patient's body or in an *in vitro* sample. Particularly preferably, the molecular target structure is localized on the surface of cells and is, therefore, hampered from floating throughout the body or *in vitro* sample. Preferably, the molecular target structure is a structure suitable to be recognized as an epitope by an antibody. Preferably, the molecular target structure is a polypeptide and/or a fatty acid, more preferably a polypeptide. Most preferably, the molecular target structure is a membrane-bound polypeptide (membrane protein) or a soluble polypeptide (extracellular protein).

The target structure-binding compound may be a high molecular weight compound of a molecular weight of 5 kDa or more or may be small molecule of a molecular weight of less than 5 kDa. Preferably, the structure-binding compound is a high molecular weight compound of a molecular weight of 5 kDa or more, more preferably of more than 10 kDa, even more preferably of more than 20 kDa, even more preferably of more than 50 kDa and even more preferably of more than 100 kDa. Exemplarily, the target structure-binding compound may be a polypeptide, a polysaccharide, a fatty acid, an eicosanoid, a steroid, a synthetical pharmaceutical agent (e.g., a small molecule drug) a synthetical polymer (e.g., methacrylate (MA), hydroxypropyl methacrylate (HPMA), polylactic acid (PLA), polyglycolic acid (PGA), polyethylene glycol (PEG), polylysine (PL)) conjugated to one or more target structure-binding moieties (e.g. polypeptide(s), fatty acid(s), eicosanoid(s), steroid(s), synthetical pharmaceutical agent(s) (e.g., small molecule drug(s)) or a combination of two or more thereof) or a combination of two or more thereof. Preferably, the target structure-binding compound is a polypeptide. More preferably, the target structure-binding compound is an antibody or a mutant or fraction thereof.

A target structure-binding compound may typically, but not necessarily, have at least two binding sites:

(i) at least one binding site for binding to the second domain of the polypeptide of the present invention; and
(ii) at least one binding site for binding to the molecular target structure.

Herein, the two binding sites may be located on different domains or on the same domain. Preferably, the two binding sites are located on different domains (e.g., an Fc fragment and Fab fragment of an antibody). Typically but not necessary, the at least one binding site for binding to the second domain of the polypeptide of the present invention is identical or similar in all target structure-binding compounds (e.g., an Fc fragment of an antibody) used with a particular polypeptide according to the present invention, whereas the binding site for binding to the molecular target structure is different in a variety of target structure-binding compounds, each binding to an individual molecular target structure. This setup enables the modular assembly of a probe.

As mentioned above, the contrast agent may be any contrast agent known in the art. Preferably, a contrast agent is an agent detectable by any means of molecular imaging known in the art. Therefore, the contrast agent in the context of the present invention may preferably serve as a means for radiocontrast agent (by X-ray attenuation) also usable in Computer Tomography (CT), and/or Magnetic Resonance Imaging (MRI) contrast agent (by MR signal enhancing). However, it may optionally also serve as ultrasound contrast agent (used in contrast-enhanced ultrasound), electron microscopy or combinations of two or more of the aforementioned. All these techniques are well-known to those skilled in the art. The contrast agent may be any contrast agent known in the art.

In a preferred embodiment, the contrast agent is a metal atom or metal ion.

In a more preferred embodiment, the contrast agent is a metal atom or metal ion, preferably wherein the metal atom or metal ion is a rare earth atom or rare earth ion or $Fe^{2+}$, $Fe^{3+}$ or $Mn^{2+}$ more preferably $Gd^{3+}$, $Dy^{3+}$, $Yb^{3+}$ or $Ho^{3+}$, in particular $Gd^{3+}$.

The first domain may bind to the contrast agent by non-covalent or covalent binding. Preferably, the first domain binds to the contrast agent by a non-covalent binding, in particular by complex formation. Therefore, particularly preferably, the contrast agent is $Gd^{3+}$ that is bound to the second domain of the polypeptide according to the present invention via complex formation. The formed complex may then also be designated as polypeptide-$Gd^{3+}$ complex.

Also the second domain may bind to the target structure-binding compound by non-covalent or covalent binding. Preferably, the second domain binds to the target structure-binding compound by non-covalent binding.

In a highly preferred embodiment, the second domain binds to the target structure-binding compound non-covalently.

As used herein, a non-covalent binding is in the broadest sense any binding not comprising covalent bonds. Typically, non-covalent binding is provoked, beside others, by one or more ionic interaction(s), one or more hydrophobic interaction(s), one or more hybrid bond formation(s) and/or one or more pi-pi electron system interaction(s).

As mentioned above, the first domain of the polypeptide according to the present invention may be any domain binding to a contrast agent. Preferably, the first domain is a calcium-binding domain.

In a preferred embodiment, the first domain comprises parvalbumin, a calcium-binding polypeptide of the EF hand family, a calcium-binding annexin polypeptide or a calcium-binding plasma membrane ATPase or a contrast agent-binding fraction or mutant of any thereof, in particular wherein the first domain comprises alpha parvalbumin or a contrast agent-binding fraction or mutant thereof.

As used herein, a calcium-binding polypeptide of the EF hand family exemplarily is calmodulin (CaM), S100, calbindin, troponin C, aequorin. As used herein, a calcium-binding annexin polypeptide preferably includes the consensus sequence G-X-G-T-X(39)-D/E.

Particularly preferably, the first domain or a contrast agent-binding fraction or mutant thereof or a pharmaceutically acceptable salt of one of the aforementioned has at least 50%, at least 60%, at least 70%, at least 80%, at least 85%, at least 90%, or at least 95% sequence and/or structure homology with or may be the polypeptide of SEQ ID NO: 7:

SMTDLLSAEDIKKAIGAFTAADSFDHKKFFQMVGLKKKSADDVKKVFHILDKDKD

GFIDEDELGSILKGFSSDARDLSAKETKTLMAAGDKDGDGKIGVEEFSTLVAES

The polypeptide domain having the sequence of SEQ ID NO: 7 represents the S55D/E59D mutant of rat alpha-parvalbumin, a high affinity $Ca^{2+}$ binding protein [3] trapping $Gd^{3+}$.

A contrast agent-binding fraction or mutant of such a peptide binds the contrast agent with an affinity that has at least 10%, at least 20%, at least 50%, at least 60%, at least 70%, at least 80%, at least 85%, at least 90%, at least 95% or even 100% of the binding affinity of the polypeptide of SEQ ID NO: 7. Preferably, a fraction of such peptide comprises at least 10, at least 20, at least 40, at least 50 or at least 60 or more consecutive amino acids of the amino acid sequence of SEQ ID NO: 7 with not more than one, not more than two, not more than three, not more than four, or not more than five single amino acid mutation(s) and/or frame shift mutation(s).

As mentioned above, the target structure-binding compound may be any compound selectively binding to a molecular target structure, wherein said compound comprises preferably a polypeptide.

In a preferred embodiment, the target structure-binding compound is an antibody or a fragment or mutant thereof binding to an antigen.

As used in the context of the present invention, the term "antibody" may be understood in the broadest sense as any type of immunoglobulin or antigen-binding fraction or mutant thereof known in the art.

Exemplarily, the antibody of the present invention may be an immunoglobulin A (IgA), immunoglobulin D (IgD), immunoglobulin E (IgE), immunoglobulin G (IgG), immunoglobulin M (IgM), immunoglobulin Y (IgY) or immunoglobulin W (IgW). Preferably, the antibody is an IgA, IgG or IgD. More preferably, the antibody is an IgG. However, it will be apparent that the type of antibody may be altered by biotechnological means by cloning the gene encoding for the antigen-binding domains of the antibody of the present invention into a common gene construct encoding for any other antibody type.

Preferably, the binding between the antibody or mutant thereof and its molecular target structure, i.e., its antigen, is a non-covalent binding. Preferably, the binding affinity of the antibody or mutant thereof to its antigen has a dissociation constant (Kd) of less than 1 $\mu$M, less than 500 nM, less than 200 nM, less than 100 nM, less than 50 nM, less than 40 nM, less than 30 nM or even less than 20 nM.

As used in the context of the present invention, the term "antibody mutant" may be understood in the broadest sense as any antibody fragment, antibody mimetic or antibody with altered sequence known in the art. The antibody mutant may have at least 10%, at least 20%, at least 50%, at least 60%, at least 70%, at least 80%, at least 85%, at least 90% or at least 95% of the binding affinity of a corresponding antibody, i.e., bear a dissociation constant (Kd) of less than 10 $\mu$M, less than 1 $\mu$M, less than 500 nM, less than 200 nM, less than 100 nM, less than 50 nM, less than 40 nM, less than 30 nM or even less than 20 nM.

As used herein, the term "antibody fragment" may be understood in the broadest sense as any fragment of an antibody that still bears binding affinity to its molecular target, i.e., its antigen. Exemplarily, the antibody fragment may be a fragment antigen binding (Fab fragment), a truncated antibody comprising one or both complementarity determining region(s) (CDR(s)) or the variable fragment (Fv) of an antibody. Preferably, the antibody fragment bears an Fc part to which the polypeptide of the present invention may bind. The antibody fragments may be of natural origin, of gene technologic origin and/or of synthetical origin.

As used herein, the term "antibody mimetic" may be understood in the broadest sense as organic compounds that, like antibodies, can specifically bind antigens and that typically have a molecular mass in a range of from approximately 3 kDa to approximately 25 kDa. Antibody mimetics may be, e.g., affibody molecules (affibodies), affilins, affitins, anticalins, avimers, DARPins, Fynomers, Kunitz domain peptides, single-domain antibodies (e.g., VHH antibodies or VNAR antibodies, nanobodies), monobodies, diabodies, triabodies, flexibodies and tandabs. The antibody mimetics may be of natural origin, of gene technologic origin and/or of synthetical origin. Optionally, the antibody may also be a CovX-body. Optionally, the antibody may also be a cameloid species antibody.

As used herein, the term "antibody with altered sequence" may be understood in the broadest sense as any antibody that has at least 50%, at least 60%, at least 70%, at least 80%, at least 85%, at least 90% or at least 95% sequence and/or structure homology with a naturally occurring antibody.

It will be understood that the terms "antibody" and "antibody mutant" as used in the context of the present invention also encompass the salts thereof. The antibody or mutant thereof, independent on its chemical nature, may optionally be dissolved in any medium suitable for storing said antibody such as, e.g., water, an aqueous buffer (e.g., a Hepes, Tris, or phosphate buffer), blood, serum, plasma, lymph or an organic solvent (e.g., dimethyl sulfoxide (DMSO), dimethylformide (DMF)). It will further be understood that the antibody or mutant thereof may be stored at conditions suitable for it, such as, e.g., in solution at room temperature, in solution in a cooled environment (e.g., at 4°C), in frozen (at -20°C), deep-frozen (e.g., -80°C, -196°C), freeze-dried and/or dry state (e.g., spray-dried) depending on the chemical nature of the antibody or the mutant thereof. Preferably, the antibody or mutant thereof has a molecular weight of more than 3 kDa, more than 5 kDa, more than 10 kDa, more than 20 kDa, more than 30 kDa, more than 50 kDa, more than 100 kDa, more than 110 kDa, more than 120 kDa, more than 130 kDa or even more than 135 kDa. Most preferably, the molecular weight of the antibody is between 135 kDa and 170 kDa. The antibody or mutant thereof according to the present invention may be of any species or origin. It may bind to any epitope(s) comprised by its molecular target structure (e.g., linear epitope(s), structural epitope(s), primary epitope(s), secondary epitope(s)). The epitope may be accessible by the antibody in the natural configuration or may be a hidden epitope (particularly detectable in denaturized polypeptides or polysaccharides). Preferably, the antibody or mutant thereof may recognize the naturally folded molecular target structure or a domain or fragment thereof. The antibody or mutant thereof may be of any origin an antibody may be obtained from such as, e.g., natural origin, a gene technologic origin and/or a synthetic origin. Optionally, the antibody may also be commercially available. The person skilled in the art will understand that the antibody may further comprise one or more posttranslational modification(s) and/or may be conjugated to one or more further structures such as labeling moieties or CPPs as exemplified in the context of the polypeptide above.

As used herein, the target structure-binding compound of the present invention, in particular an antibody or mutant thereof, may comprise a target structure-binding domain and any other domain typically found in an antibody.

In a more preferred embodiment, the target structure-binding compound comprises an Fc fragment or mutant thereof that is binding to the second domain.

As used herein, the term "Fc fragment" may be understood in the broadest sense as commonly understood by those skilled in the art, i.e. as fragment crystallizing of an antibody. The Fc may be the Fc of any antibody known in the art such as e.g., an Fc of an IgA, IgD, IgG, IgM or IgE antibody. A fragment thereof may be any truncated form thereof of at least four, at least 10, at least 20, at least 30, at least 40, at least 50, at least 60, at least 70, at least 80, at least 90 or at least 95 consecutive amino acids thereof. A mutant thereof may be any form with altered amino acid sequence thereof having at least four %, at least 10%, at least 20%, at least 30%, at least 40%, at least 50%, at least 60%, at least 70%, at least 80%, at least 90% or at least 95% sequence and/or structure homology to the corresponding naturally occurring Fc fragment.

Preferably, this Fc fragment can be bound by the second domain of the polypeptide of the present invention. This second domain may have any structure or sequence binding to an Fc fragment known in the art.

In one exemplarily preferred embodiment, the second domain comprises an Fc fragment-binding polypeptide domain of protein A of *Staphylococcus aureus.*

Particularly preferably, the Fc fragment-binding polypeptide domain or a pharmaceutically acceptable salt thereof has at least 50%, at least 60%, at least 70%, at least 80%, at least 85%, at least 90%, or at least 95% sequence and/or structure homology with or may be the polypeptide of SEQ ID NO: 8:

VDNKFNKEQQNAFYEILHLPNLNEEQRNAFIQSLKDDPSQSANLLAEAKKLNDAQ
APK

The polypeptide domain having the sequence of SEQ ID NO: 8 represents a mutant of the B-domain of *Staphylococcus aureus* protein A called Z-domain [1]. This polypeptide is capable of binding commercially available therapeutic IgG antibodies via their constant region.

The polypeptide may be provided by any means known in the art. It may be provided by a commercial or a non-commercial supplier, may be provided by a biological sample (in particular a blood sample), may be provided by any means of genetic engineering known in the art or may be provided by chemical means such as peptide synthesis (e.g., solid phase peptide synthesis).

Highly preferably, at least one protein A domain and a rat alpha-parvalbumin are linked with another by a linker polypeptide. Even more preferably, the Z-domain of protein A and S55D/E59D rat alpha-parvalbumin are conjugated via a linker.

Most preferably, the polypeptide of the present invention is a fusion polypeptide wherein the C-terminus of the Z-Domain is conjugated with the N-terminus of Parvalbumin via a decaglycine. This polypeptide is denoted Zalbin.

In a preferred embodiment, the polypeptide is obtainable by genetic engineering, preferably without synthetic chemical modifications.

As used herein, the term "genetic engineering" may be understood in the broadest sense as any biotechnological technique available in the art to prepare genetic constructs and expressing these in a host cell or in a cell-free expression system in order to obtain the polypeptide of the present invention. As used herein, "without chemical modifications" means that beside the expression of the polypeptide in a host cell or in a cell-free expression system and potential posttranslational modification(s) occurring thereby, the polypeptide is not subjected to any manmade chemical steps.

Highly preferably, the sequence of the polypeptide of the present invention comprises:

(a) at least one first domain having at least 50%, at least 60%, at least 70%, at least 80%, at least 85%, at least 90% or at least 95% sequence and/or structure homology with a polypeptide of SEQ ID NO: 7 or being a polypeptide having an amino acid sequence of SEQ ID NO: 7; and

(b) at least one second domain having at least 50%, at least 60%, at least 70%, at least 80%, at least 85%, at least 90% or at least 95% sequence and/or structure homology with a polypeptide of SEQ ID NO: 8 or being a polypeptide having an amino acid sequence of SEQ ID NO: 8.

Particularly preferably, the polypeptide of the present invention or a pharmaceutically acceptable salt thereof may have at least 50%, at least 60%, at least 70%, at least 80%, at least 85%, at least 90%, or at least 95% sequence and/or structure homology with the polypeptide of SEQ ID NO: 9 or may be a polypeptide having an amino acid sequence of SEQ ID NO: 9:

VDNKFNKEQQNAFYEILHLPNLNEEQRNAFIQSLKDDPSQSANLLAEAKKLNDAQ
APKGGGGGGGGGGSMTDLLSAEDIKKAIGAFTAADSFDHKKFFQMVGLKKKSAD
DVKKVFHILDKDKDGFIDEDELGSILKGFSSDARDLSAKETKTLMAAGDKDGDGK
IGVEEFSTLVAES

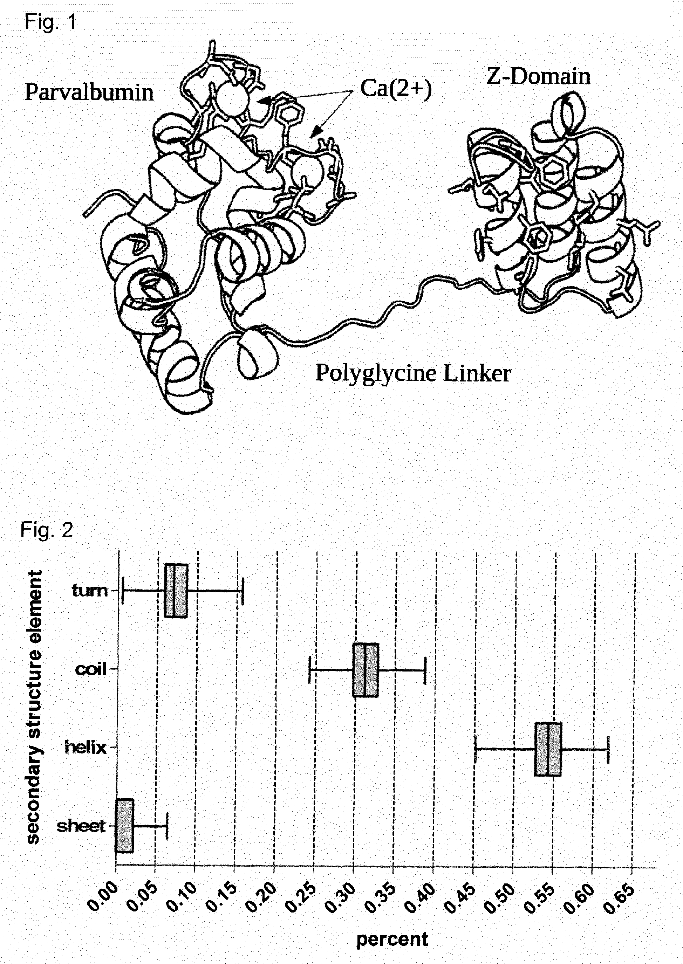

The polypeptide having an amino acid sequence of SEQ ID NO: 9 represents the Zalbin protein as described in the examples and depicted in Figure 1. As described above, in the Zalbin polypeptide, the C-terminus of the Z-Domain is conjugated with the N-terminus of Parvalbumin via a decaglycine linker.

The invention also comprises a functional fraction of such peptide comprising at least 20, at least 30, at least 40, at least 50, at least 60, at least 70, at least 80, at least 100 or at least 150 or more consecutive amino acids of the amino acid sequence of SEQ ID NO: 9 with not more than one, not more than two, not more than three, not more than four, or not more than five, not more than six, not more than seven, not more than eight, not more than nine or not more than ten single amino acid mutation(s) and/or frame shift mutation(s).

The polypeptide of the present invention may also be used to form a probe for molecular imaging comprising the two or more binding partners thereof, i.e., at least one contrast agent and at least one target structure-binding compound binding to a second domain of said polypeptide.

Therefore, in a second aspect, the present invention relates to a probe for molecular imaging comprising the following components:

> (a) at least one polypeptide according to the present invention; and
> (b) at least one contrast agent binding to a first domain of said polypeptide,
> and optionally further comprising:
> (c) at least one target structure-binding compound binding to a second domain of said polypeptide.

The probe will typically be formed by contacting the aforementioned components with another by any way known in the art. Preferably, the probe is formed, thus, the above-referenced components are contacted with another prior to being administered to a patient *in vivo* or to an *in vitro* sample. More preferably, the components are contacted with another prior to being administered in a liquid medium such as, e.g., liquid medium comprising water, an aqueous buffer and/or an organic solvent. As used in the context of the present invention, the term "patient" may be understood in the broadest sense as any living being, which is preferably any animal, more preferably a mammal including human, in particular a human being.

Alternatively, the probe may be formed upon administration of the components independently into a patient *in vivo* or into a sample *in vitro,* wherein all three components may be administered independently from another (i.e., the at least one polypeptide, the at least one contrast agent and the at least one structure-binding compound) or two of them are combined with another (e.g., the at least one polypeptide and the at least one contrast agent forming a polypeptide-contrast agent complex) and the third component (e.g., the at least one target structure-binding compound) is administered to the patient or *in vitro* sample independently or is present in the patient's body or *in vitro* sample inherently (e.g., an antibody in the patient's blood or lymph or an *in vitro* sample).

The at least one target structure-binding compound binding to a second domain of said polypeptide may also be present inherently in the patient's body or in an *in vitro* sample. Therefore, the probe may then be free of any added heterologous (i.e., allogene or xenogene) target structure-binding compound and may comprise at least one polypeptide according to the present invention and at least one contrast agent binding to a first domain of said polypeptide contacted with another before administration to a patient. The probe may then be administered to a patient or an *in vitro* sample in which the at least one target structure-binding compound binding to a second domain of said polypeptide (most typically an antibody) is present inherently. Therefore, most preferably, the probe comprising at least one polypeptide according to the present invention and at least one contrast agent binding to a first domain of said polypeptide may form a non-covalent complex with an autologous antibody inside the patient.

As indicated above, the target structure-binding compounds will typically bear one or more binding site(s) suitable for binding to the second domain of the polypeptide of the present invention which will typically but not necessary be identical or similar in all target structure-binding compounds (e.g., an Fc fragment of an antibody) and one or more binding site(s) suitable for binding to the molecular target structure which are variable. This setup enables the modular assembly of a probe comprising one kind of polypeptide according to the present invention, one kind of contrast agent, but a variety of target structure-binding compounds binding to different molecular target structures.

Most preferably, the at least one target structure-binding compound may be added to the other components *ex vivo.* Then, the assembly of a probe is a modular assembly in which in particular the polypeptide according to the present invention may be combined with a variety of target structure-binding compounds as long as said target structure-binding compounds bind to a second domain of the polypeptide of the present invention.

The present invention further refers to the use of a contrast agent, in particular $Gd^{3+}$, in a method for the diagnosis of a patient suffering from or being at risk of developing at least one of the physiological or pathological condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration, wherein said contrast agent is administered together with the polypeptide of the present invention.

Accordingly, the present invention further refers to a contrast agent, in particular $Gd^{3+}$ for use in a method for the diagnosis of a patient suffering from or being at risk of developing at least one of the physiological or pathological condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration, wherein said contrast agent is administered together with the polypeptide of the present invention.

The present invention further refers to the use of an antibody in a method for the diagnosis of a patient suffering from or being at risk of developing at least one of the physiological  or pathological condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration, wherein said antibody is administered together with the polypeptide of the present invention.

Accordingly, the present invention further refers to an antibody for use in a method for the diagnosis of a patient suffering from or being at risk of developing at least one of the physiological or pathological condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration, wherein said antibody is administered together with the polypeptide of the present invention.

Highly preferably, the probe according to the present invention comprises the following components:

(a) a polypeptide comprising at least one first domain having at least 50%, at least 60%, at least 70%, at least 80%, at least 85%, at least 90% or at least 95% sequence and/or structure homology with a polypeptide of SEQ ID NO: 7 or being a polypeptide having an amino acid sequence of SEQ ID NO: 7 and at least one second domain having at least 50%, at least 60%, at least 70%, at least 80%, at least 85%, at least 90%, or at least 95% sequence and/or structure homology with a polypeptide of SEQ ID NO: 8 or being a polypeptide having an amino acid sequence of SEQ ID NO: 8;
(b) $Gd^{3+}$ binding to said first domain; and
(c) an antibody binding to said second domain of said polypeptide.

Particularly preferably, the probe according to the present invention comprises the following components:

(a) a polypeptide having at least 50%, at least 60%, at least 70%, at least 80%, at least 85%, at least 90%, or at least 95% sequence and/or structure homology with the polypeptide of SEQ ID NO: 9 or being a polypeptide having an amino acid sequence of SEQ ID NO: 9;
(b) $Gd^{3+}$ binding to a first domain of said polypeptide; and
(c) an antibody binding to a second domain of said polypeptide.

It will be understood that the probe may optionally be dissolved in any medium suitable for storing said probe such as, e.g., water, an aqueous buffer (e.g., a Hepes, Tris, or phosphate buffer), blood, serum, plasma, lymph or an organic solvent (e.g., dimethyl sulfoxide (DMSO), dimethylformide (DMF)). It will further be understood that the probe may be stored at conditions suitable for it, such as, e.g., in solution at room temperature, in solution in a cooled environment (e.g., at 4°C), in frozen (at -20°C), deep-frozen (e.g., - 80°C, -196°C), freeze-dried and/or dry (e.g., spray-dried) state.

As used in the context of the present invention, the term "molecular imaging" may be understood in the broadest sense as any method suitable for determining the localization and/or local concentration of a molecular target structure. As used herein, molecular imaging is preferably medicinal imaging. Molecular imaging may also be visualization.

The probe of the present invention may also be designated as antibody-directed proteinogenic contrast agent. Highly preferably, this probe is a gadolinium (III) based $T_1$ contrast agent for magnetic resonance imaging (MRI).

In a preferred embodiment, molecular imaging is selected from the group consisting of: magnetic resonance imaging; and

radiography, in particular computer tomography.

The aforementioned techniques are well-known to those skilled in the art. As used herein, magnetic resonance (MR) spectroscopy is preferably Magnetic Resonance Imaging (MRI).

Alternatively or additionally, molecular imaging may also be microscopy (e.g., bright field microscopy (light microscopy), fluorescence microscopy, laser scanning microscopy, two-photon fluorescence microscopy, electron microscopy), Fluorescence Molecular Imaging (FMI), Fluorescence Molecular Imaging (FMI), in particular Fluorescence Molecular Tomography (FMT), Optical Imaging, Single Photon Emission Computed Tomography (SPECT), Positron Emission Tomography (PET), Ultrasound Tomography (UT), fluorescence microscopy, laser scanning microscopy, two-photon microscopy, fluorescence energy transfer (FRET) based methods, fluorescence correlation spectroscopy (FCS), fluorescence cross-correlation spectroscopy (FCCS), electron microscopy or combinations of two or more thereof. All these techniques are well-known to those skilled in the art. Moreover, the present invention may be used in any other immunological method known in the art, such as e.g., fluorescence activated cell sorting (FACS), by Western Blot, by ELISA.

The person skilled in the art will understand that the present invention also relates to any means for producing the polypeptide and probe of the present invention. As mentioned above, the polypeptide is preferably obtainable by genetic engineering.

Therefore, a further aspect of the present invention relates to a nucleic acid molecule encoding for a polypeptide according to the present invention.

The term "nucleic acid molecule" as used herein may be understood in the broadest sense as any molecule suitable for conveying genetic information. Preferably, the nucleic acid molecule is deoxyribonucleic acid (DNA) (double stranded DNA (dsDNA), single stranded DNA (ssDNA)), ribonucleic acid (RNA), (single-stranded RNA (ssRNA), double-stranded RNA (dsRNA)), or as a DNA analog such as, e.g., peptide nucleic acid (PNA), morpholino, glycol nucleic acid (GNA), threose nucleic acid (TNA) or methylated DNA. More preferably, the nucleic acid molecule is DNA, in particular double-stranded DNA. It will be understood that a nucleic acid molecule may also be a salt, in particular a pharmaceutically acceptable salt, of one of the aforementioned.

Preferably, the nucleic acid molecule further comprises a start codon, a stop codon and/or a promoter enabling the translation thereof. More preferably, the nucleic acid molecule further comprises a start codon, a stop codon and a promoter enabling the translation thereof.

Accordingly, in a further aspect, the present invention relates to a vector comprising the nucleic acid molecule according to the present invention.

As used in the context of the present invention, the term "vector" may be understood as any vehicle enabling the translation of the nucleic acid molecule and expression of the polypeptide of the present invention in a cell or in a cell-free expression system. The vector may be any vector known in the art such as, e.g., a linear vector, a circular vector, a viral vector or a bacterial vector. Preferably, the vector comprises one or more linear or circular nucleic acid molecule(s) (e.g., a plasmid) or is a viral vector. It will be understood that the vector may also be a salt, in particular a pharmaceutically acceptable salt, of one of the aforementioned. Optionally, the vector may be a linear expression cassette that may or may not be integrated in the genome of the target cell. However, it may be understood that every other vector may also be used. In a vector, the nucleic acid molecule encoding for the polypeptide according to the present invention is typically flanked by starting and stop codons and upstream of this coding region the vector comprises a promoter. Optionally, the vector further comprises one or more enhancer sequences. As used herein, the term "promoter" may be understood in the broadest sense as a region of DNA that facilitates the transcription of the gene encoding for the polypeptide according to the present invention. The promoter may be located near the gene, and may be located on the same strand and upstream. Upstream means that it is located towards the 5' region of the sense strand encoding for the polypeptide. The promoter may be a homologous promoter or a heterologous promoter. The term "expressed" as used throughout the present invention may be understood in the broadest sense as the conversion of genetic information into a polypeptide strand.

In principle, the polypeptide of the present invention may be translated and expressed by any means known in the art, i.e., in any cell or cell-free expression system. Preferably, the vector is translated and expressed in a host cell.

Therefore, a still further aspect of the present invention relates to a host cell comprising the vector according to the present invention.

The host cell in the context of the present invention may be any cell suitable for comprising the vector of the present invention, preferably wherein the host cell is also suitable for translating the gene encoding the polypeptide according to the present invention into the respective messenger RNA (mRNA) and expressing said polypeptide. The host cell may be any cell known in the art. Exemplarily, the host cell may be a prokaryotic or a eukaryotic cell. It may be a bacterial cell (e.g., an *E. coli* cell), an animal cell (e.g., a mammal cell or an insect cell), a fungal cell (e.g., a yeast cell) or a plant cell. Preferably, the host cell is a bacterial or an animal cell. The host cell may also be a cell in which a vector as described above is inserted by technical means. In this context, the vector may be inserted into the cell by any means known in the art, and may be used in combination with or without transfecting agent(s) (e.g., lithium acetate, polyethylene imine (PEI), fugene, LT-1, JetPEI, transfectamine, lipofectamine, UptiFectin, PromoFectin, GenePORTER, Hilymax, carbon nanofibres, carbon nanotubes cell-penetrating peptides (CPPs), protein transduction domains (PTDs), liposomes, DEAE-dextran, dendrimers), with or without electroporation, or with or without a gene gun, with or without optical transfection with or without gene electrotransfer, with or without impalefection, with or without magnetofection, and/or with or without magnet assisted transfection.

Optionally, the host cell may be selected by means of using any selection marker known in the art such as, e.g., antibiotic resistance and/or beta-galactosidase conversion. The person skilled in the art will immediately know how to use such selection markers.

Optionally, the host cells, independent on how it is generated, may additionally be screened by one or more method(s) generally well-known in the art such as, e.g., one or more immunological method(s) (e.g., flow cytometry, enzyme-linked immunosorbent assay (ELISA), Western Blot, co-precipitation assay), one or more nucleic acid-based method(s) (e.g., DNA gel electrophoresis, Southern Blot, Northern Blot) and/or one or more functional assays (e.g. binding studies of the polypeptide). The host cells may be cultured at conditions suitable for these cells to survive and, preferably, to proliferate. The host cell may also be stored in frozen, deep-frozen, freeze-dried and/or dry state (e.g., spray dried) depending on the biological nature of the host cell.

In order to generate the polypeptide according to the present invention, the host cells may be cultured at conditions enabling the generation of said polypeptide. Optionally, the polypeptide may then be recovered from the culture broth.

The polypeptide, probe, nucleic acid and/or vector of the present invention or a pharmaceutical salt of one thereof and/or host cell of the present invention may also form part of a diagnostic composition.

Accordingly, in a further aspect, the present invention relates to a diagnostic composition comprising:

(a) at least one polypeptide according to the present invention, at least one probe according to the present invention, at least one nucleic acid molecule according to the present invention, at least one vector according to the present invention and/or at least one host cell according to the present invention;
(b) at least one contrast agent binding to a first domain of the polypeptide according to the present invention; and
(c) a pharmaceutically acceptable carrier.

As used herein, the term "pharmaceutically acceptable carrier" may refer to any substance that may support the pharmacological acceptance of the diagnostic composition comprising a polypeptide, a probe, a nucleic acid molecule, a vector and/or a host cell according to the present invention. The diagnostic composition may be pharmaceutically formulated in a dry form (e.g., as a powder, a tablet, a pill, a capsule, a chewable capsule, etc.) or a liquid (e.g., a spray, a syrup, a juice, a gel, a liquid, a paste, an injection solution, etc.).

Preferably, a pharmaceutically acceptable carrier may be a liquid carrier or a solid carrier. Exemplarily, a pharmaceutically acceptable carrier may comprise a solvent with no or low toxicity such as, e.g., water, an aqueous buffer (e.g., a Hepes, Tris, or phosphate buffer, a  phosphate buffer), a pharmaceutically acceptable organic solvent (e.g., dimethyl sulfoxide (DMSO), ethanol, vegetable oil, paraffin oil) or a combination of two or more thereof.

Furthermore, the pharmaceutically acceptable carrier may contain one or more detergents, one or more foaming agents (e.g., sodium lauryl sulfate (SLS)/ sodium doceyl sulfate (SDS)), one or more colouring agents (e.g., $TiO_2$, food colouring), one or more vitamins, one or more salts (e.g., sodium, potassium, calcium, zinc salts), one or more humectants (e.g., sorbitol, glycerol, mannitol, propylenglycol, polydextrose), one or more enzymes, one or more preserving agents (e.g., benzoic acid, methylparabene), one or more texturing agents (e.g., carboxymethyl cellulose (CMC), polyethylene glycol (PEG), sorbitol), one or more emulsifiers, one or more bulking agents, one or more glacing agents, one or more separating agents, one or more antioxidants, one or more herbal and plant extracts, one or more stabilizing agents, one or more polymers (e.g., hydroxypropyl methacrylamide (HPMA), polyethylene imine (PEI), carboxymethyl cellulose (CMC), polyethylene glycol (PEG)), one or more uptake mediators (e.g., polyethylene imine (PEI), dimethyl sulfoxide (DMSO), a cell-penetrating peptide (CPP), a protein transduction domain (PTD), an antimicrobial peptide, etc.) one or more antibodies, one or more sweeteners (e.g., acesulfame, an acesulfame salt (e.g., acesulfame potassium (acesulfame K), aspartame, cyclamate, saccharin, a saccharin salt (e.g., saccharin sodium (saccharin Na)), alitame, neotame, sucralose, dulcin, salt of aspartame-acesulfame, sorbitol, stevia, glycerol, inulin, mannitol, isomalt, maltitol, malto-oligosaccharide, lactitol, xylitol, glucin, neohesperidin dihydrochalcone, P-4000, brazzein, curculin, erythritol, glycyrrhizin, hydrogenated starch hydrolysates, luo han guo, mabinlin, miraculin, monatin, monellin, osladin, pentadin, tagatose, thaumatin), one or more counterstain dyes (e.g., fluorescein, fluorescein derivatives, Cy dyes, an Alexa Fluor dyes, S dyes, rhodamine, quantum dots, etc.), one or more homeopathic ingredients one or more gustatory substances and/or one or more fragrances. The diagnostic composition will typically be formed by contacting the aforementioned component with another by any way known in the art. Preferably, the diagnostic composition is formed, thus, the above-referenced components are contacted with another prior to being administered to a patient.

Preferably, a diagnostic composition according to the present invention comprises:

(a) a polypeptide according to the present invention;

(b) Gd$^{3+}$ binding to a first domain of said polypeptide; and
(c) a pharmaceutically acceptable carrier.

In a more preferred embodiment, the diagnostic composition further comprises at least one component selected from the group consisting of:

(d) at least one target structure-binding compound;
(e) at least one nucleic acid molecule encoding for the target structure-binding compound;
(f) at least one vector comprising the nucleic acid molecule encoding for the target structure-binding compound; and
(g) at least one host cell suitable for expressing the target structure-binding compound.

Highly preferably, the diagnostic composition according to the present invention comprises a probe according to the present invention and a pharmaceutically acceptable carrier.

Highly preferably, the diagnostic composition according to the present invention comprises:

(a) a polypeptide comprising at least one first domain having at least 50%, at least 60%, at least 70%, at least 80%, at least 85%, at least 90%, at least 95% sequence and/or structure homology with a polypeptide of SEQ ID NO: 7 or being a polypeptide having an amino acid sequence of SEQ ID NO: 7 and at least one second domain having at least 50%, at least 60%, at least 70%, at least 80%, at least 85%, at least 90% or at least 95% sequence and/or structure homology with a polypeptide of SEQ ID NO: 8 or being a polypeptide having an amino acid sequence of SEQ ID NO: 8;
(b) Gd$^{3+}$ binding to said first domain;
(c) an antibody binding to said second domain; and
(d) a pharmaceutically acceptable carrier.

Particularly preferably, the diagnostic composition according to the present invention comprises:

(a) a polypeptide having at least 50%, at least 60%, at least 70%, at least 80%, at least 85%, at least 90% or at least 95% sequence and/or structure homology with the polypeptide of SEQ ID NO: 9 or may be a polypeptide having the sequence of SEQ ID NO: 9;
(b) Gd$^{3+}$ binding to a first domain of said polypeptide;
(c) an antibody binding to a second domain of said polypeptide; and
(d) a pharmaceutically acceptable carrier.

The diagnostic composition according to the present invention may be used for diagnosis purposes.

Accordingly, in a further aspect, the present invention relates to the polypeptide, the probe, the nucleic acid, the vector, the host cell and/or the diagnostic composition according to the present invention for use in a method for the diagnosis of a patient suffering from or being at risk of developing at least one of the physiological or pathological condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration.

Most preferably, the polypeptide of the present invention forms a complex with a contrast agent (e.g., Gd$^{3+}$) and a target structure-binding compound (e.g., a specific antibody). This complex binds to the molecular target structure of the target structure-binding compound, e.g., when the target structure-binding compound is an antibody, the epitope of said antibody. Preferably, the presence of the molecular target structure in a particular concentration or location indicates the particular physiological or pathological condition of interest.

Most preferably, the molecular target structure is a stationary target structure (e.g., a membrane protein) typical for the physiological or pathological condition of interest. Then, the complex comprising the polypeptide of the present invention, a contrast agent (e.g., Gd$^{3+}$) and a target structure-binding compound (e.g., a specific antibody) accumulates at certain positions in the patient's body or in the *in vitro* sample. The contrast agent (e.g., Gd$^{3+}$) may then be visualized (e.g., by molecular imaging). This may provide the desired diagnostic information on the physiological or pathological condition of interest.

As used herein, a physiological condition may be understood in the broadest sense as any state of the patient that may occur in nature. A pathological condition may be any condition of a non-healthy patient who suffers from a disease with or without noticing symptoms. Therefore, it will be understood that such pathological condition can be, but is not necessarily associated with pain or any other recognizable problems, but may also be unnoticed by the patient and/or by the medicinal expert. Preferably, the patient is being at risk of or has a particular pathological condition and/or is or has been subjected to a treatment of said pathological condition, most preferably wherein said pathological condition is cancer with or without metastases.

Preferably, the conditions associated with a local alteration of the molecular target structure concentration may be the change in a polypeptide concentration on cellular surfaces, i.e., in the cell membrane composition. Exemplarily, the concentration of a particular receptor may be increased on a contrast cell in comparison to the respective healthy cell of the same cell type. Therefore, the present invention may also be used to visualize a tumor and/or metastases thereof.

Alternatively or additionally, the conditions associated with a local alteration of the molecular target structure concentration may be plaque formation comprising one or more molecular target structure(s) recognized by the target structure-binding compound, in particular wherein said plaque formation is the formation of polypeptide plaques.

The diagnostic composition may be administered to the patient by any means known in the art such as, e.g., by injection or by oral, nasal or transdermal administration. Administration may be local administration (e.g., intratumorally (*i.t.*)) or systemic administration (e.g., intravenously (i.v.), intraarterially (*i.a.*)*,* intraperitoneally (*i.p.*), intramusculary (*i.m.*), subcutaneously (*s.c.*)). Preferably, the pharmaceutical administration is administered by injection, more preferably systemic injection, in particular intravenous injection.

When the nucleic acid or the vector according to the present invention is used in the aforementioned diagnostic method, cells of the patient may express the polypeptide of the present invention in the patient *in vivo.* When the host cell according to the present invention is used in the aforementioned diagnostic method, the host cells may express the polypeptide of the present invention in the patient *in vivo.*

In a preferred embodiment, the diagnostic composition is administered to the patient prior to or concomitantly with detecting a signal by molecular imaging.

More preferably, the diagnostic composition is administered to the patient prior to detecting a signal by molecular imaging. Then, the diagnostic composition is administered to the patient followed by a waiting time. Exemplarily, this waiting time may be few seconds, few minutes, at least 30 min, at least 1 h, at least 2 h, at least 3 h, at least 4h, at least 5 h, at least 6 h, at least 12 h, a least 24 h or even longer.

In the diagnostic composition according to the present invention, the polypeptide may be administered (preferably in the form of a polypeptide-Gd$^{3+}$ complex) as well as the compound selectively binding to a molecular target structure (e.g. an antibody) or said polypeptide (preferably in the form of a polypeptide-Gd$^{3+}$ complex) may be administered alone and said compound selectively binding to a molecular target structure (e.g. an antibody) is present in the patient inherently.

In a preferred embodiment, the polypeptide according to the present invention is administered concomitantly with, prior to or subsequent to the administration of the compound selectively binding to a molecular target structure.

More preferably, the polypeptide according to the present invention is administered concomitantly the administration of the compound selectively binding to a molecular target structure.

In more a preferred embodiment, a probe according to the present invention is formed in *vitro* prior to being administered to the patient.

Therefore, as mentioned, most preferably, the diagnostic composition according to the present invention comprises a probe according to the present invention and a pharmaceutically acceptable carrier. Alternatively, the diagnostic composition may comprise a polypeptide-Gd$^{3+}$ complex and a pharmaceutically acceptable carrier and the target structure-binding compound (e.g., an antibody) binding to said polypeptide is administered independently in a second diagnostic composition comprising said target structure-binding compound and a pharmaceutically acceptable carrier.

The results obtained from molecular imaging may be analyzed by any analysis method known in the art.

In a preferred embodiment, the diagnostic composition is administered to the patient prior to an incubation period allowing the probe to bind to its molecular target structure followed by molecular imaging and subsequent comparison of the acquired result with a standard.

As mentioned above said incubation period may be few seconds, few minutes, at least 30 min, at least 1 h, at least 2 h, at least 3 h, at least 4h, at least 5 h, at least 6 h, at least 12 h, a least 24 h or even longer. As used herein, comparison of the acquired result with a standard may be comparison of the results obtained from the diagnosis of the patient with previously obtained results for a healthy patient, preferably with the same or comparable imaging settings and/or comparison of the results obtained from the diagnosis of the patient with previously obtained results for a patient in the articular physiological or pathological state of interest, preferably with the same or comparable imaging settings. These results may be used in order to determine the physiological or pathological state of interest of the patient. Optionally, the operator may or may not deduce a particular medicinal treatment therefrom.

The diagnostic composition of the present invention may optionally be administered to the patient once, twice, three times, four times, five times or even more often. Exemplarily, between these administrations, there may be an incubation period of few seconds, few minutes, at least 30 min, at least 1 h, at least 2 h, at least 3 h, at least 4h, at least 5 h, at least 6 h, at least 12 h, a least 24 h or even longer.

The polypeptide, probe, nucleic acid molecule, vector and/or diagnostic composition may also form part of a kit, preferably a commercially available kit.

Therefore, a further aspect of the present invention relates to a kit comprising at least one polypeptide according to the present invention, at least one probe according to the present invention, at least one nucleic acid molecule according to the present invention encoding for said polypeptide, at least one vector according to the present invention encoding for said polypeptide, at least one host cell according to the present invention suitable for expressing said polypeptide and/or at least one diagnostic composition according to the present invention; and further comprising at least one of the components selected from the group consisting of:

(a) at least one contrast agent binding to a first domain of said polypeptide;
(b) at least one target structure-binding compound binding to the second domain of said polypeptide;
(c) at least one nucleic acid molecule encoding for the compound of (b);
(d) at least one vector comprising the nucleic acid molecule of (c);
(e) at least one host cell comprising the vector of (d);
(f) at least one carrier; and
(g) a user manual.

Highly preferably, the kit according to the present invention comprises a polypeptide comprising at least one first domain having at least 50%, at least 60%, at least 70%, at least 80%, at least 85%, at least 90% or at least 95% sequence and/or structure homology with a polypeptide of SEQ ID NO: 7 or being a polypeptide having an amino acid sequence of SEQ ID NO: 7 and at least one second domain having at least 50%, at least 60%, at least 70%, at least 80%, at least 85%, at least 90% or at least 95% sequence and/or structure homology with a polypeptide of SEQ ID NO: 8 or being a polypeptide having an amino acid sequence of SEQ ID NO: 8;
$Gd^{3+}$ binding to said first domain; and
a user manual,
and optionally further comprises an antibody binding to a second domain of said polypeptide and/or a carrier.

Highly preferably, the kit according to the present invention comprises a polypeptide having at least 50%, at least 60%, at least 70%, at least 80%, at least 85%, at least 90% or at least 95% sequence and/or structure homology with the polypeptide of SEQ ID NO: 9 or a polypeptide having an amino acid sequence of SEQ ID NO: 9;
$Gd^{3+}$ binding to a first domain of said antibody; and
a user manual,
and optionally further comprises an antibody binding to a second domain of said polypeptide and/or a carrier.

As used herein, a carrier may be any substance or substantial mixture for improving the properties of the composition. Preferably, a carrier comprises at least one selected from water, an aqueous buffer or an organic solvent. The carrier may or may not be pharmaceutically acceptable. Most preferably, the carrier is a pharmaceutically acceptable carrier.

As used herein, a user manual may, exemplarily, comprise information on how to use the polypeptide, probe, nucleic acid molecule, vector and/or diagnostic composition in the detection and diagnostic methods described herein. Exemplarily, it may comprise information on how to generate the polypeptide and/or probe. Further, when it comprises a diagnostic composition it may comprise information on how to use said diagnostic composition, i.e., when and how to administer it, how to perform the measurements, i.e., the molecular imaging, how to perform the analysis of the results and/or for which physiological and pathological conditions said diagnostic composition is intended to be used.

In a highly preferred embodiment, the kit comprises:

a probe according to the present invention; and
a user manual.

Optionally, said kit may further comprise one or more carrier(s), in particular one or more pharmaceutically acceptable carrier(s), which may or may not be premixed with the probe according to the present invention. The probe may, therefore, be dissolved in a carrier or may be delivered as a dried or freeze-dried powder or as dried or freeze-dried pellets or tablets. Optionally, the kit may also comprise a probe immobilized on a solid support. Most preferably, in the kit, the probe is dissolved in a pharmaceutically acceptable carrier.

In a further alternative highly preferred embodiment, the kit comprises:

a polypeptide according to the present invention;
$Gd^{3+}$ binding to a first domain of said polypeptide; and
a user manual,
and the target structure-binding compound binding to the second domain of said polypeptide (e.g., an antibody) is not comprised in said kit, but is rather to be admixed by the user him or herself.

Herein, the polypeptide and $Gd^{3+}$ are typically present in the form of a polypeptide and $Gd^{3+}$-complex. However, alternatively, the polypeptide and $Gd^{3+}$ may also be present in the kit separated from another. The user may ad a large variety of different target structure-binding compounds as long as these are binding the second domain of the polypeptide according to the present invention.

In an alternative highly preferred embodiment, the kit comprises:

> a polypeptide according to the present invention;
> $Gd^{3+}$ binding to a first domain of said polypeptide;
> a target structure-binding compound binding to the second domain of said polypeptide, in particular an antibody; and
> a user manual,
> wherein at least one of said polypeptide, said $Gd^{3+}$ and said target structure-binding compound is not premixed with the others.

In a still further alternative highly preferred embodiment, the kit comprises:

> a polypeptide according to the present invention; and
> a user manual,
> and $Gd^{3+}$ binding to a first domain of said polypeptide and the target structure-binding compound binding to the second domain of said polypeptide (e.g., an antibody) are not comprised in said kit, but are rather to be admixed by the user him or herself.

In a still further alternative highly preferred embodiment, the kit comprises:

> a vector according to the present invention; and
> a user manual.

As already mentioned above, the polypeptide according to the present invention may be obtained by any means, preferably by genetic engineering.

Therefore, in a further aspect, the present invention also relates to a method for producing the polypeptide according to the present invention, said method comprising the following steps:

> (i) providing a host cell according to the present invention,
> (ii) culturing said host cell under conditions that allow said host cell to express the polypeptide according to the present invention; and
> (iii) recovering said polypeptide from said culture.

The person skilled in the art will know how to culture the cells in order to maintain their viability and to stimulate expression of the polypeptide. A constitutive promoter typically does not require such stimulation, whereas an inducible promoter requires stimulation of a transcription factor.

As used herein, the term "recovering said polypeptide" as used herein may be understood in the broadest sense as any means for obtaining the polypeptide from the culture. As the host cells will typically be cultured in a culture broth, also designated as culture medium, the polypeptide is present in a mixture comprising several salts, sugars, buffer agents, amino acids, growth factors and cells. Recovering of the polypeptide means that a polypeptide previously being part of this mixture is taken from the cell culture. Preferably, recovering the polypeptide may further comprise isolating the polypeptide from the culture broth. Then, the polypeptide concentration is increased. It will, however, be understood that also an isolated polypeptide may optionally be dissolved in a liquid medium comprising further components.

Recovering and isolation may be performed by any mean known in the art such as, e.g., by any chromatographic means (e.g., by affinity chromatography (e.g., based on a solid support bearing immobilized binding partners of the first or the second domain of said polypeptide, a solid support bearing a binding moiety binding to a tag structure comprised by said polypeptide (e.g., a poly-HIS tag) and/or a solid support bearing immobilized antibodies against said polypeptide), gel permeation chromatography (GPC) (molecular sieve chromatography, size exclusion chromatography), hydrophobicity chromatography, hydroxyapatite chromatography, fast protein liquid chromatography (FPLC), high-performance liquid chromatography (HPLC) (e.g., reversed phase high-performance liquid chromatography (RP-HPLC), in particular RP-HPLC based on C4-C24 gel material), ultra high performance liquid chromatography (UPLC) (e.g., reversed phase ultra high-performance liquid chromatography (RP-UPLC) ), in particular RP-UPLC based on RP-C4, RP-C6, RP-C8, RP-C10, RP-C12, RP-C14, RP-C16, RP-C18, RP-C20, RP-C22, RP-C24 gel material), salting in, salting out, immunoprecipitation, co-immunoprecipitation, magnetic beads conjugated to a binding partner of the polypeptide of the present invention, gel electrophoresis (e.g., sodium dodecyl sulfate polyacryl gel electrophoresis (SDS-PAGE), blue native gel electrophoresis) or a combination of two or more thereof. Should the employed purification method denaturize the tertiary and/or secondary structure of the polypeptide, the purification method denaturize may preferably be followed by a renaturalization step (e.g., dissolving in a suitable buffer, buffer exchanging GPC, native gel electrophoresis, dialysis), wherein the polypeptide may re-fold in the functional tertiary structure again.

The polypeptide may, optionally, also be isolated from the cell culture. Preferably, an isolated polypeptide may constitute for at least 20 %(w/w), at least 40 %(w/w), at least 50 %(w/w), at least 60 %(w/w), at least 70 %(w/w), at least 80 %(w/w), at least 90 %(w/w) or even 100 %(w/w) of the total polypeptide content. It will, however, be understood that also an isolated polypeptide may, optionally, be dissolved in a liquid medium comprising further components. The polypeptide of the present invention once recovered or even isolated may, therefore, subsequent to isolation also be mixed with other polypeptides again leading to a lower content of the polypeptide of the present invention in the final composition such as, e.g., present in a diagnostic composition or a kit.

Purity of the peptide may, exemplarily, be determined by means of any chromatographic means (e.g., by affinity chromatography, GPC, hydrophobicity chromatography, hydroxyapatite chromatography, FPLC, HPLC (e.g., RP-HPLC), UPLC (e.g., RP-UPLC)), gel electrophoresis (e.g., SDS-PAGE or blue native gel electrophoresis), mass spectrometry (MS) (e.g., matrix-assisted laser desorption ionization mass spectrometry (MALDI-MS), electrospray mass spectrometry (ESI-MS)) or a combination of two or more thereof (e.g., MS-MS coupling, HPLC-MS, HPLC-MS-MS, UPLC-MS, UPLC-MS-MS).

The recovered polypeptide may optionally also be conjugated to at least one labeling moiety and/or at least one solid support by any chemical and/or biological means known for such conjugations known in the art. The person skilled in the art will know several conjugation methods such as, e.g., the formation of amide bonds by means of an active ester e.g., succinimidyl ester) or the conjugation of a thiol moiety with a maleimide. Conjugation of the polypeptide according to the present invention to a solid support may be used for providing functional nano- or microbeads for diagnosis of a patient *in vivo* or for *in vitro* imaging, affinity chromatography beads and/or microarrays.

As described above, the probe according to the present invention may be used in a diagnostic composition for use in a method for diagnosing a patient *in vivo*. It will, therefore, be understood by a person skilled in the art that the probe according to the present invention is likewise usable in any method for detecting the localization and/or the local concentration of a molecular target structure, in particular also in an *in vitro* method.

Accordingly, a still further aspect of the present invention relates to a method for detecting the localization and/or the local concentration of a molecular target structure comprising the following steps:

(i) contacting an object with a probe according to the present invention;
(ii) incubating said probe in contact with said object under conditions allowing said probe to bind to its molecular target structure;
(iii) performing molecular imaging of said object;
(iv) comparing the acquired result of step (iii) with a standard.

Said method may be performed *in vivo or in vitro.*

As used herein, an object may be any object which is suitable for being observed by molecular imaging. Preferably, the object comprises biological material. As used herein, the term "biological material" comprises material from a living organism or a dead organism that has lived before such as, not more than 30 min before, not more than 1 h before, not more than 2 h before, not more than 6 h before, not more than 12 h before, not more than 24 h before, not more than 1 week before, not more than 1 month before, not more than a year before, or more than a year before. Such organism is preferably but not necessarily a multicellular organism, more preferably a living or dead animal body, even more preferably a living or dead mammal body including human, most preferably a living or dead human body.

As described in the context of the diagnostic composition above, in a preferred embodiment, a local alteration in the concentration compared to the standard indicates a specific physiological or pathological condition, wherein, in a more preferred embodiment, the specific physiological or pathological condition is a condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration.

Accordingly, a further aspect of the present invention relates to the use of a polypeptide according to the present invention, a probe according to the present invention, a nucleic acid molecule according to the present invention, a vector according to the present invention, a host cell according to the present invention, a diagnostic composition according to the present invention and/or a kit according to the present invention for detecting the localization and/or the local concentration of a molecular target structure.

Herein, the use may be *use in vivo* and/or *in vitro.*

As described in the context of the diagnostic composition and the method for detecting the localization and/or the local concentration of a molecular target structure above, in a preferred embodiment, a local alteration in the concentration compared to a standard indicates a specific physiological or pathological condition, wherein, in a more preferred embodiment, the specific physiological or pathological condition is a condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration.

If not otherwise defined, all numerical specifications are to be considered as approximate values allowing typical deviations of several percent. The person skilled in the art will also understand that the present invention further also encompasses the salts of any of the compounds described herein, in particular any pharmaceutically acceptable salts.

The following examples as well as the accompanying figures are intended to provide illustrative embodiments of the present invention described and claimed herein. These examples are not intended to provide any limitation on the scope of the invented subject-matter.

**Brief description of the drawings**

**Figure 1** shows a cartoon representation of Zalbin. Parvalbumin (left) bound with two calcium ions (spheres) connected with the Z-domain (right) via a decaglycine linker. The Fc binding residues of the Z-domain according to Deisenhofer [18] are shown as sticks.

**Figure 2** shows the boxplot of the secondary structure elements over the MD simulations.

**Figure 3** shows a histogram of the distances between the Z-Domain and Parvalbumin.

**Figure 4** shows a histogram of the minimal distances between the Fc binding atoms of the Z-Domain and the surface atoms of parvalbumin.

**Figure 5** shows a number of water molecules in the first coordination sphere of the Ca(2+) ions of parvalbumin in percent. The black and white bars refer to the Ca(2+) ions in the structure 1S3P with residue numbers 178 and 179, respectively.

**Figure 6** shows the CD spectrum of Zalbin.

**Figure 7** shows the titration of Zalbin-Atto-465 with the monoclonal IgG antibody Cetuximab.

**Figure 8** shows the confocal microscopic analysis of the complex Cetuximab:Zalbin-D72C-Atto 594 binding to the EGF receptor located in the cell membrane of A431 cells.

**Figure 9** shows the titration of 3 $\mu$M TbCl3 and 8 $\mu$M Zalbin with NTA. Luminescence of Terbium (III) was recorded. The curve was normalized and inverted prior to fitting with a Hill equation.

**Figure 10** shows the competition titration of 4 $\mu$M Zalbin and 10 $\mu$M TbCl3 with GdCl3 and CaCl2, respectively. Luminescence of Terbium(III) was recorded.

**Figure 11** shows the relaxometric properties of Zalbin:$(Gd^{3+})$2 at three different field strengths employing an inversion recovery TSE experiment. A diluted solution of rising concentrations of Zalbin:$(Gd^{3+})$2 was investigated to find the limiting concentration which still produces a visible contrast towards the buffer control (0 $\mu$M). The picture is displayed with an inversion time TI which zeroes the signal of the buffer control (appears black).

**Figure 12** shows the stability of Zalbin in 50% FCS over time. Aliquots were taken after distinct times and investigated on degradation of Zalbin employing a Schägger-Jagov gel. M: Mark 12.

**Figure 13** shows the thermal stability of Zalbin (b), Zalbin:$(Gd^{3+})$2 (d) and the single domains parvalbumin (a) and Z-domain (c).

**Examples**

Example 1

Materials and Methods

If not otherwise stated all chemicals were purchased either from Carl Roth (Karlsruhe, Germany) or Sigma Aldrich (Steinheim, Germany). All spectroscopic measurements (except those using a NanoDrop) were performed using semi-micro or micro quartz cuvettes from Hellma Analytics (Müllheim, Germany).

Modeling

All structural templates were taken from the Protein Data Bank (PDB) [4]. Three dimensional models of Zalbin were generated with the MODELLER 9.1 [5] software, using as templates the Z-Domain (PDB entry 1q2n [3]) and the calcium bound structure of Parvalbumin (PDB entry 1s3p [6]). The C-terminus of the Z-Domain was linked to the N-terminus of Parvalbumin with a decaglycine. In total, we modelled 50 structures.

Clustering and secondary structure prediction

We used a cluster algorithm [7] to group the models according to a RMSD cut-off of 1.7 A. For one structure in each cluster, we performed a molecular dynamics (MD) simulation. DSSP [8] was used to analyse the secondary structure.

Molecular dynamics simulations

MD simulations were performed in the NPT ensemble at 300K and atmospheric pressure using GROMACS 4.0 [9] with the GROMOS 43a1 force field [10] and SPC/E water. Temperature and pressure was stabilized at 300 K by Nose-Hoover thermostat and 1 atm by Parinello-Rahman barostat, respectively. Periodic boundary conditions with triclinic boxes were applied with a minimum of 1.0 nm distance between protein and faces of box. Residues were assumed to be protonated according to their normal states at pH 7. $Na^+$ and $Cl^-$ ions were added to neutralize the system at an ionic strength of 0.15 mol/l. The Particle Mesh Ewald method was used to compute electrostatic interactions boundary conditions. Bonds involving hydrogen atoms were constrained using the SHAKE algorithm [11], allowing for a time step of 2 fs. Structures were energy minimized and equilibrated by molecular dynamics for 1 ns. Snapshots of the trajectories were saved every 100 ps. In total, we simulated the system for 200 ns. The last 5 ns of all trajectories were analyzed with the toolbox provided by GROMACS, especially g_rms for root-mean-square-deviations (RMSDs) and g_rmsf for root-mean-square-fluctuations (RMSFs). Due to the physical-chemical similarity of $Ca^{2+}$ and $Gd^{3+}$, we used the default $Ca^{2+}$ parameter.

Distance analysis and water coordination analysis

To analyse the dynamic behaviour of the system, we calculated the distance between the centre of mass (COM) of both domains using the g_dist program available in the Gromacs package. g_dist was also used to calculate the number of water molecules that are present in the first coordination sphere of Ca(2+). We determined the number of water molecules in a distance of 3.4 Å to the calcium ion, the maximum distance between the calcium ion and a water molecule in the first coordination sphere according to Playinski et al. [12]. To check for intramolecular interactions, we calculated the minimum distance between the atoms of both domains using the g_mindist program of the Gromacs package.

Cloning and mutation

The gene encoding the two-domain protein Zalbin was commercially synthesised and subcloned in a derivative of a pET41b expression vector containing GST and a PreScission cleavage site, both located N-terminal of the multiple cloning site (Geneart, Regensburg, Germany). The cloning sites used were *Apa*I and *Bam*HI. The single Z- and parvalbumin-domains were subcloned in the same vector by first amplifying the respective genes from the full length construct via PCR. The primer used for Z-domain subcloning were 5' CACACAGGGCCCGTGGATAACAAATTTAACAAAGAACAGC-3' (SEQ ID NO: 1) for forward ApaI cloning and 5' GGTTGGGGATCCATTATTTCGGCGCCTGCGCATCGT 3' (SEQ ID NO: 2) for reverse BamHI cloning. The primer used for rat S55D/E59D-alpha-parvalbumin subcloning were 5' CACACAGGGCCCAGCATGACCGATCTGCTGAGCGC 3' (SEQ ID NO: 3) for forward *Apa*I cloning and 5' GGTTGGGGATCCATTAGCTTTCCGCCACCAGGG 3' (SEQ ID NO: 4) for reverse *Bam*HI cloning. Mutation of Zalbin yielding a D72C mutant was performed using a Quik-Change® kit (Stratagene). The respective primers used for mutation were forward 5' GGCAGCATGACCTGTCTGCTGAGC 3' (SEQ ID NO: 5) and reverse 5' GCTCAGCAGACAGGTCATGCTGCC 3' (SEQ ID NO: 6).

Expression and purification of recombinant Zalbin

Recombinant Zalbin was expressed as GST fusion protein. Expression was performed in *E. coli* BL21(DE3) T1r cells. For that, after cells were grown up to OD 0.6 in $2\times$ YT medium at 37 °C protein expression was induced with 0.2 mM IPTG. Expression was performed for another 5 h at 29 °C. Bacteria pellets were suspended in 20 mM Tris, 50 mM NaCl, pH 7.4 and cells were cracked via lysozyme treatment for 1 h at 4 °C following sonication at room temperature. The supernatant of subsequent ultracentrifugation was bound to a 20 ml GSTrap column (GE Healthcare) using a BioRad FPLC at 4 °C. Bound protein was washed with three buffers to remove unwanted components. First, a high salt buffer consisting of 20 mM Tris, 500 mM NaCl, pH 7.4 was used to remove unspecifically bound proteins from the column. A buffer consisting of 20 mM Tris, 150 mM NaCl, 70 mM EDTA, pH 7.4 was used to remove potentially bound $Ca^{2+}$ ions from the metal ion binding sites of the parvalbumin domain. Another buffer consisting of 20 mM Tris, 150 mM NaCl, 0.2 mM ATP, pH 7.4 was used to remove potentially bound bacterial chaperones. Elution was performed using 20 mM Tris, 150 mM NaCl, 20 mM glutathione, pH 7.4.

Eluted fusion protein was concentrated and glutathione was removed via dialysis using a 30 kDa MWCO centricon (Millipore) by repeated centrifugation and refilling of the centricon. Afterwards, cleavage with PreScission protease was performed at 4 °C over night. Zalbin was purified by first removing GST via a 20 ml GSTrap column. The flow-through was collected and concentrated again. Gelfiltration using a Superdex 75 26/60 column (GE Healthcare) was performed to remove aggregates and degraded protein. The buffer used for GST removal as well as for gel filtration contained 20 mM Tris, 150 mM NaCl, pH 7.4. Purified protein was aliquoted, shock frozen and stored at -20 °C. The D72C mutant of Zalbin and the separate parvalbumin domain were purified in the same way, however 1 mM DTT was added to all buffers for purification of Zalbin-D72C. The separate Z-domain was also purified as GST fusion protein, only missing the washing step with EDTA. Due to the architecture of the PreScission cleavage site the two amino acids GP remain at the N terminus of the protein.

MALDI-MS

Proteins were desalted using Supel-Tips C18 (Sigma) and eluted with 50:50 (v/v) acetonitrile : 0.1 % TFA in water. Matrix solution was prepared by dissolving 7.6 mg 2',5'-dihydroxy acetophenone in ethanol and adding 125 ul of a solution containing 18 mg/ml di-ammonium hydrogen citrate in water. Proteins were spotted on a Ground steel target plate (Bruker Daltonics) using the dried droplet method. For that, 2% TFA in water were mixed with the desalted protein and matrix solution in a ratio of 1:1:1 (v/v). The spot was dried at room temperature. Mass analysis was carried out using an Autoflex speed MALDI-TOF (Bruker Daltonics).

Analytical Gelfiltration

Gelfiltration experiments were performed using a Superdex 75 10/300 GL or a Superdex 75 16/26 column (GE Healthcare) and a BioRad FPLC at 4 °C. For calibrating the column Alpha-Amylase (porcine pancreas), Ovalbumin (Sigma), GAPDH from rabbit muscle (Sigma), Chymotrypsinogen A from bovine pancreas and Lysozyme (Fluka) were used. Runs were performed at 0.5 ml/min in 20 mM Tris, 500 mM NaCl, pH 7.4 and 20 mM Tris, 200 mM NaCl, 3 M guanidine hydrochloride, pH 7.4.

Protein concentration determination

Protein concentrations of stock solutions were determined by measuring the absorbance of the solution at 280 nm or 258 nm, respectively, using a NanoDrop ND 1000 (peqlab). Absorbance was measured at least three times and averaged following concentration determination using an extinction coefficient of 1490 $M^1cm^1$ for Zalbin as well as for the Z-domain and 217315 $M^1cm^1$ for Cetuximab. Parvalbumin concentrations were determined by measuring the absorbance at 258 nm and using an extinction coefficient of 1512 $M^1cm^1$, which is the sum of the absorbances of eight phenylalanine residues. It was calculated with an absorbance of 189 $M^1cm^1$ at 258 nm for one phenylalanine [13]. Exact protein concentrations of samples where CD spectra were recorded from were measured using a Cary 100 UV/Vis spectrophotometer.

CD spectroscopy

CD spectroscopy was performed using a JASCO J815 CD spectrometer. For recording of spectra Zalbin was diluted in 20 mM Natrium phosphate buffer, pH 7.4 at a final concentration of approximatly 0.15 mg/ml and using a 1 mm cuvette. CD spectra were recorded from 200 - 260 nm. Further parameters were data pitch: 0.2 nm, scanning mode: continuous, scanning speed: 100 nm/min, response time: 0.5 s, bandwidth: 2 nm. 15 scans were averaged for each spectrum. Spectra were recorded at room temperature. Data processing was performed by separately shifting the blank and the protein spectrum to zero for the wavelengths 250 - 260 nm and subsequent subtraction of the blank spectrum from the protein spectrum. Afterwards, the mdeg units of the spectrum were transformed to $\Delta\varepsilon$ MRW units using a mean residue weight (MRW) of 107.091 Da for Zalbin and the spectroscopically determined concentration of the protein in mg/ml units. These data processing steps were performed using unpublished software (Steve Martin, NIMR London ). Secondary structure contents of Zalbin were calculated using the programs CDSSTR, Contin 11 and Selcon 3. The complete range of secondary structure contents calculated by the three programs is displayed in table 1.

Melting curves of Zalbin and the single domains were performed by diluting the proteins in 20 mM HEPES, 20 mM NaCl, pH 7.4 at a final concentration of 0.09 mg/ml and using a 2 mm cuvette. Recording parameters used were data pitch: 0.1 °C, delay time: 180 s, bandwidth: 1 nm, response time: 8 s, temperature slope: 0.5°C/min for melting and 1°C/min for cooling.

Labelling of Zalbin

N-terminal labelling of Zalbin as well as labelling of the cysteine residue of Zalbin-D72C was performed using NHS-Ester and maleimide derivatives of Atto dyes respectively (Atto-tec, Siegen, Germany). Labelling was performed according to the Atto-tec protocols for amine and thiol reactive dyes respectively. The chosen labelling strategy was 1 h at room temperature and subsequent separation of the protein-dye complex from unbound dye using two PD-10 columns (GE Healthcare) in a row. Corrected protein concentrations of the protein-dye complexes and labelling efficiencies were determined spectroscopically using a Cary 100 UVNis spectrophotometer. If not used immediately, labelled protein was shock frozen and stored at -20°C.

Fluorescence anisotropy titrations

Fluorescence anisotropy measurements were performed using a Cary eclipse fluorescence spectrometer (Agilent Technologies) equipped with a polariser. Approximately 120 nM of Zalbin N terminally labelled with Atto-465 was titrated with the monoclonal antibody Cetuximab by recording the fluorescence anisotropy signal of Atto-465. The buffer used was 20 mM Tris, 150 mM NaCl, pH 7.4. Measurements were performed using following parameters: 440 nm for excitation of Atto-465 and 525 nm for emission, excitation slit width: 10 nm, emission slit width: 20 nm, PMT voltage: 600 V, temperature: 20°C, G-factor for Atto-465: 1.6848. Data points of the titration were recorded in the kinetic mode using an average time of 7 s and a recording time of at least 10 min for each point.

Cell culture and fluorescence microscopy

Binding of the complex of Cetuximab and Zalbin-D72C-Atto594 to the cell membrane bound EGF receptor of A431 cells was visualised by fluorescence microscopy using a Leica SP5 confocal microscope. For that, A431 cells were grown to a density of 105 - 106 cells/cm2 on $\mu$-Slide 8 well plates (Ibidi). Afterwards, medium was removed and cells were incubated with a mixture of Cetuximab and Zalbin-D72C-Atto594, Cetuximab alone, Zalbin-D72C-Atto594 alone or buffer without any protein for 30 minutes at room temperature. The used buffer was 25 mM HEPES, 150 mM NaCl, 4 mM KCl, pH 7.4. A431 cells are viable in this buffer for days. Protein concentrations were 7.8 $\mu$M Cetuximab and 14 $\mu$M Zalbin-D72C-Atto594 in HEPES buffer. Finally, unbound protein was removed by 5 washing steps with HEPES buffer and cells were left in this buffer containing 10% FCS during microscopy. Atto-594 was excited with a 594 nm laser and an intensity of 50%. Emission was detected between 605 - 750 nm with a PMT voltage between 800 volts. Auto fluorescence of cells was measured by exciting with a 405 nm  diode laser and detecting fluorescence between 417 - 502 nm. The laser intensity used was 70% and the PMT voltage 820 V.

Pictures were recorded with a resolution of 8 bit, a size of 1024 x 1024 pixel, a line average of 8 and a scanning speed of 200 pixel/sec. Z-stacks were recorded with a stack thickness of 0.17 $\mu$M, a size of 1024 x 1024 pixel, a line average of 2 and a scanning speed of 400 pixel/sec. Image processing concerning brightness and contrast adjustments were done using the program Image J.

Luminescence measurements and metal titrations

Luminescence measurements were performed using a Cary eclipse fluorescence spectrometer. Terbium (III) in complex with Zalbin was excited via energy transfer from a phenylalanine between the EF and CD metal binding sites of the parvalbumin domain. The excitation wavelength used for this was 258 nm. Luminescence emission was detected at 543 nm ($^5D_4 \rightarrow {}^7F_5$ transition) with a delay time of 0.15 ms, a gate time of 4.5 ms and a total decay time of 200 ms. The excitation and emission slit widths used were 10 nm and 20 nm respectively. The PMT voltage was 800 V. Titrations were carried out at 20 °C. Each titration step was measured in kinetic mode with an average time of 7 s over a time scale of 10 minutes.

Affinity determinations of $Tb^{3+}$ to the EF and CD site of the parvalbumin domain were done in 20 mM Tris, 150 mM NaCl, pH 7.4. Each titration step was pipetted separately and all batches were incubated for three days at room temperature to establish equilibrium between the complexes Zalbin:$(Tb^{3+})_2$ and NTA:$Tb^{3+}$. The curve was normalised and inverted prior to fitting with a Hill equation. Using the fitted apparent affinity of NTA:$Tb^{3+}$ and the real binding affinity of this complex of $5.6 \times 10^{-12}$ M [14], the binding affinity of Zalbin:$(Tb^{3+})_2$ was calculated according to equation:

$$K_D{}^{Zalbin} = K_D{}^{NTA} \times [Zalbin] / (K_{app} - K_D{}^{NTA}) \text{ (equ. 1)}$$

where $K_{app}$ is the fitted apparent $K_D$ of NTA. This equation is a transposed form of an equation derived for calculating real binding constants using $K_{app}$ and [competitor] from competition titrations [15]:

$$K_D{}^{titrator} = K_{app} \times K_D{}^{competitor} / (K_D{}^{competitor} + [competitor]) \text{ (equ. 2)}$$

where in our case NTA is the titrator and Zalbin the competitor.

Competition titrations where $Tb^{3+}$ bound to Zalbin was displaced by either Gadolinium (III) or Calcium (II) were conducted in 20 mM Tris, 150 mM NaCl, 15 mM Bis-Tris, pH 7.4. The presence of Bis-Tris, which is able to chelate metal ions with low affinity, prevented adsorption of the excess $Tb^{3+}$ ions to the quartz surface of the cuvettes. Otherwise $Tb^{3+}$ would slightly be excited via energy transfer from the glass surface. The affinity of $Gd^{3+}$ and for comparison of $Ca^{2+}$ were measured using a competition assay where 4 $\mu$M Zalbin and 10 $\mu$M $Tb^{3+}$ were titrated with $Gd^{3+}$ or $Ca^{2+}$ respectively. Data were fitted using a hyperbolic equation for competition titrations [16]:

$$I_{Tb3+} = (I_{Tb3+max} \times K_{app} / (K_{app} + [M])) + I_{Tb3+min} \text{ (equ. 3)}$$

with $I_{Tb3+}$ being the $Tb^{3+}$ luminescence intensity and [M] the concentration of $Gd^{3+}$ or $Ca^{2+}$ respectively. The real binding constants of Zalbin and $Gd^{3+}/ Ca^{2+}$ were calculated using equation 2.

Nonlinear regression

Nonlinear regression for all titrations was conducted using the program Graphpad prism (Graphpad Software).

Relaxometry

Relaxometric analysis of the complex of Zalbin and two $Gd^{3+}$ ions was carried out at three different field strengths, 1.5 T, 3 T and 7 T, and at room temperature. Imaging was performed at all three field strengths using a whole-body MRI scanner (Magnetom Aera, Skyra and 7 T; Siemens Healthcare Sector, Erlangen, Germany) capable of 45 mT/m (70 mT/m at 7 T) gradient strength and 200 mT/m/s slew rate. The buffer used was 20 mM HEPES, 150 mM NaCl, 4 mM KCl, pH 7.4. Samples were put in a $4 \times 6$ well TC-Plate (Greiner bio-one) at a volume of 2.9 ml each. Concentrations of 20, 10, 5, 1, 0.5 and 0 $\mu$M Zalbin were used each containing a slightly under saturated double amount of $Gd^{3+}$ (39.5, 19.7, 9.9, 1.97, 0.99 and 0 $\mu$M).

The plate was positioned within the RF coil. A 16-channel receive wrist coil was used at 1.5 T and 3 T (Siemens Healthcare Sector), while at 7 T a 32-channel transmit/receive head coil (Nova Medical, New York, USA) was used. For the determination of $T_1$ relaxation time constants, inversion recovery turbo spin echo (IR-TSE) sequences with the following parameters were used:

inversion times (TI) between 20 and 2500 ms; repetition time/echo time (TR/TE) 12500/6.8 ms; field of view (FoV) read 180 mm; FoV phase 100.0%; frequency resolution 384; phase resolution 100%; bandwidth 395 Hz/Px; slice thickness 3 mm, nominal flip angle 180 degrees, turbo-factor 11.

The resolution of the images recorded is calculated from FOV/Matrix in READ x PHASE and the slice thickness, so 0.5 x 0.5 x 3 $mm^3$ equalling 0.75 $mm^3$. The inversion time zeroing the MRI signal (sample appears black) was used for calculating the $T_1$ relaxation time by

$$T_1 = - TI(0) / \ln(0.5).$$

The longitudinal relaxation time constant $T_1$ was calculated for each individual contrast agent at all measured concentrations and field strengths [17].

$T_1$ relaxivity $r_1$ in units of $s^1 mM^1$ further was calculated according to

$$(1/T_1 - 1/T_1^{\text{buffer}})/c(Gd^{3+})$$

and was averaged over the three wells containing 20, 10 and 5 $\mu$M Zalbin. The relaxation rate constants ($R_1$) were calculated according to the equation $R_1 = 1/T_1$. The relaxivities ($r_1$) were calculated using

$$r_1 = (1/TI(c) - 1/TI(0))/c$$

where, TI(c) is the relaxation time constant at concentration c; TI(0) is the relaxation time constant of the solvent without contrast agent and c is the concentration of a given contrast agent. The TI(0) values found for the three Zalbin concentrations were 300, 500, 800 ms for 1.5 T, 500, 800 and 1100 ms for 3 T and 900, 1350 and 1600 ms for 7 T.

Results

MD simulations show secondary structure conservation and high flexibility between both domains

A cartoon representation of Zalbin is depicted in Figure 1. The clustering of the modeled structures results in five clusters. The root mean square deviation between each cluster can be seen in table 1. For one structure of each cluster we performed a MD simulation. Table 1 summarizes the secondary structure analysis of the original PDB structures by using DSSP. The DSSP analysis of the MD trajectories shows secondary structure conservation over the simulations (Figure 2) which is in line with the results in table 2.

**Table 1.** Root mean square deviation matrix (in A) of representative structures in each cluster.

| Cluster | 1 | 2 | 3 | 4 | 5 |
|---|---|---|---|---|---|
| 1 | 0.00 | 9.22 | 11.52 | 14.69 | 7.81 |
| 2 | | 0.00 | 13.80 | 16.12 | 5.54 |
| 3 | | | 0.00 | 8.22 | 10.90 |
| 4 | | | | 0.00 | 14.50 |
| 5 | | | | | 0.00 |

**Table 2.** Calculated and measured secundary structure elements from DSSP and CD spectroscopy, respectively.

| | helix | sheet | turn | coil (unordered) |
|---|---|---|---|---|
| DSSP | 64.3% | 2.4% | 9.6% | 25.8% |
| CD spectroscopy | 61.8 - 64.7% | 1.4 - 3.7% | 7.1 - 12.1% | 23.7 - 24.7% |

Herein, the calculated secondary structure does not include the polyglycine linker which refers to 3% of the total protein. The DSSP calculation is based on the structures 1s3p and 1q2n available in the PDB. The helical portion contains 3-10, alpha and pi helical elements and the coiled structure contains loop and high curvature regions.

The dynamic behaviour of the system is illustrated in Figure 2 as a histogram of the center of mass distance between both domains. The large range shows that the decaglycine linker allows the molecule to act very felixible. The felxibility is assumed to be good for the rapid exchange with water molecules in the outer coordination shell. This is crucial for the transfer of the paramagnetic effect of $Gd^{3+}$.

Intramolecular contacts

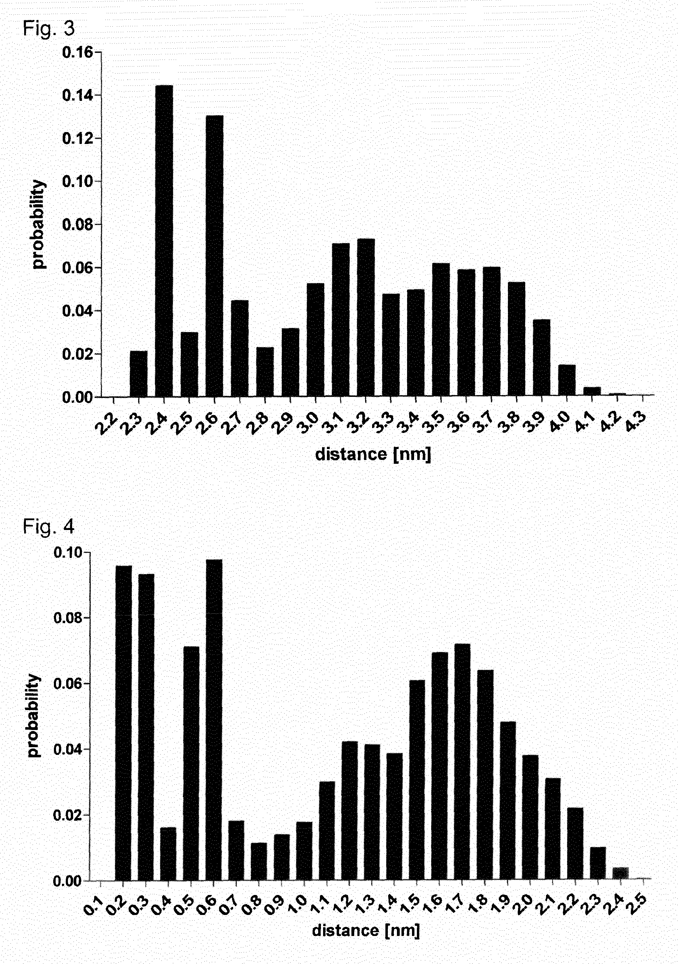

A precondition for its function as targeted contrast agent is that none of the functional sites (Fc-binding site, $Gd^{3+}$ binding site) is masked by another part of the molecule. To check for intramolecular contacts between atoms of the Z-domain involved in Fc-binding and surface atoms of parvalbumin, we performed a minimum distance analysis (Figure 4). The Fc domain binding residues were selected according to Deisenhofer [11] and include: Phe5, Gln9, Gln10, Asn11, Phe13, Tyr14, Leu17, Asn28, Ile31, Gln32 und Lys35.

Figure 3 shows that the Fc domain binding atoms of the Z-domain and the surface atoms of Parvalbumin form intramolecular contacts in the distance range from 0.23 nm to 0.28 nm.

This is due to contributions from one of the five MD simulations in which Phe13 and Gln10 in helix A of the Z-domain contacts surface atoms of the parvalbumin domain. Since these contacts involve only two peripheral amino acids and the rest of the Fc binding site remains accessible this is not likely to prevent binding to IgG antibodies.

Water in the first $Ca^{2+}$ coordination sphere

$Gd^{3+}$ exerts a paramagnetic effect on nearby water protons causing them to relax [19]. The intensities of MRI signals depend on the proton density and on the longitudinal (1/T1) and transverse relaxation rates (1/T2) of the water protons [20]. The protons in the first coordination sphere of $Gd^{3+}$ rapidly relax and the fast exchange of this water molecule with the bulk water results in transfer of the paramagnetic effect of $Gd^{3+}$ to the surrounding water protons [20].

Therefore, it is important that the $Gd^{3+}$ complexing ligands allow water molecules to enter into the first coordination sphere of the metal ion, and by rapid exchange with water in the outer coordination shell ensure the relaxation effect [21].

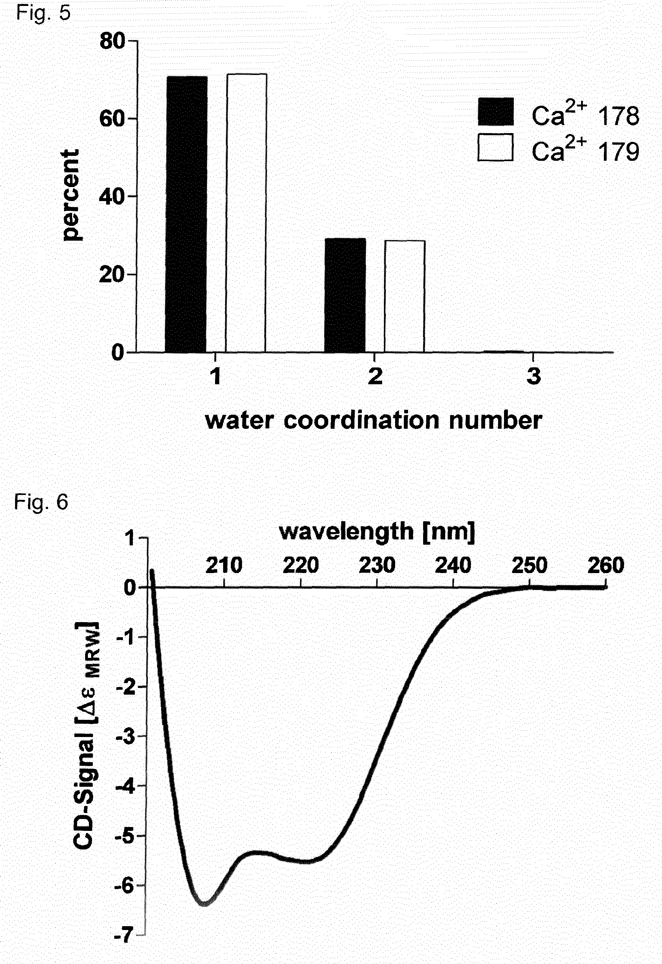

In Figure 5 we show the number of water molecules in the first coordination sphere of $Ca^{2+}$ during the MD simulations. The main water coordination number is one, which is comparable with the coordination number of $Gd^{3+}$ in clinically used chelates [20]. In about one third of the simulation time, the water coordination number increased to two water molecules, which should lead to better contrast [22]. This is also in accordance with the experimental derived number of water molecules in the first coordination sphere of lanthanide ions in parvalbumin published by Horrocks et al. [23].

The results on stability and water accessibility of the cations suggest that Zalbin has some properties that could make it an interesting candidate for a contrast agent.

Monomeric Zalbin shows structural integrity in physiological solution

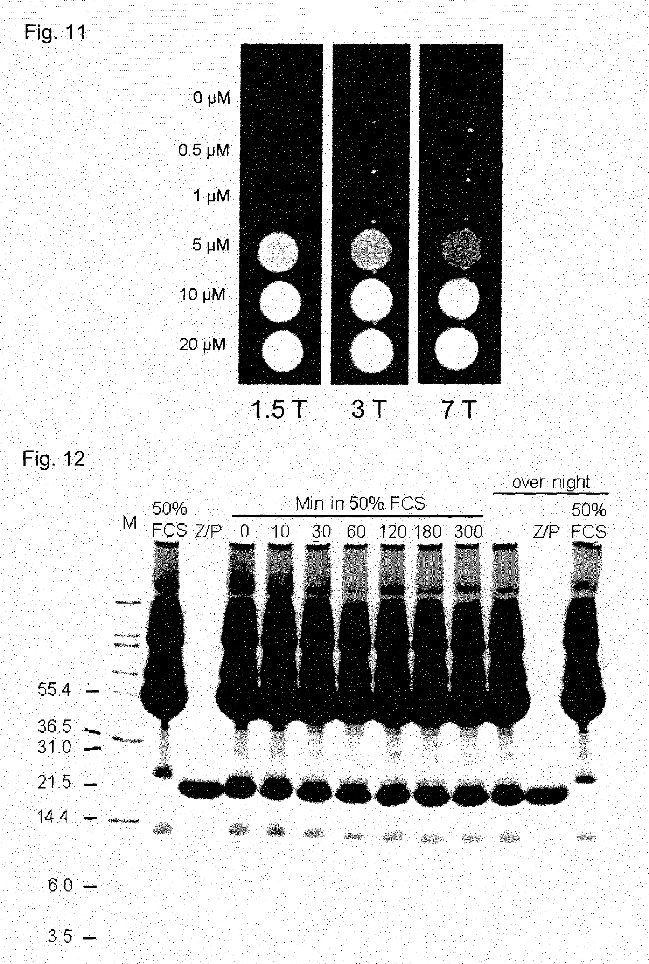

MALDI-MS analysis of the recombinant expressed and purified fusion construct Zalbin comprising the Z-domain from *staphylococcus aureus* and a S55D/E59D double-mutant of rat alpha-Parvalbumin (denoted Zalbin) provided a mass of 19156.1 Da for M + H⁺, which is in accordance with the theoretically expected value of 19156.3 Da for M + H⁺ (error: 10.44 ppm). Correct folding of Zalbin was investigated by CD spectroscopy (figure 11). The secondary structure content calculated from the resulting CD spectrum is in agreement with the secondary structure content predicted by DSSP analysis of the single domains employing their pdb files (Figure 2, Table 2). Analytical gelfiltration at pH 7.4 revealed a  molecular weight of about 32 kDa for Zalbin deviating significantly from a monomeric (19.2 kDa) or dimeric (38.3 kDa) state. The Z-domain forms trimers in solution under these conditions which is why gelfiltration was repeated at pH 7.4 and partially denaturing conditions, where the Z-domain is monomeric. As Zalbin showed the same elution profile as under native conditions the monomeric state of the fusion construct could be approved.

Integrity of Z-domain function

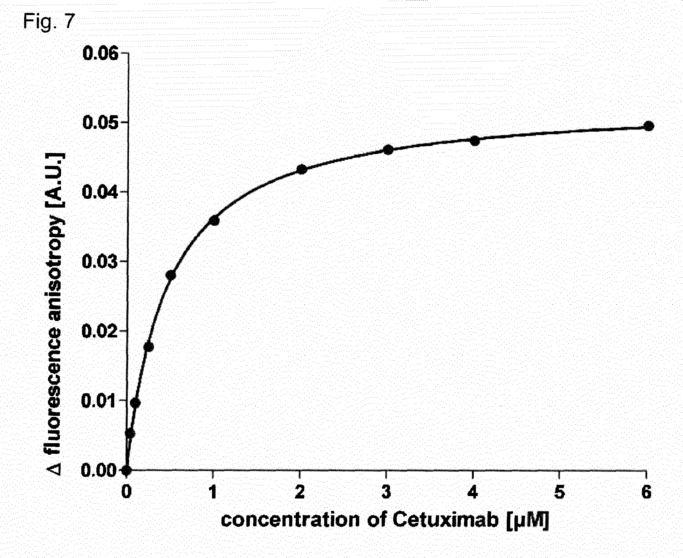

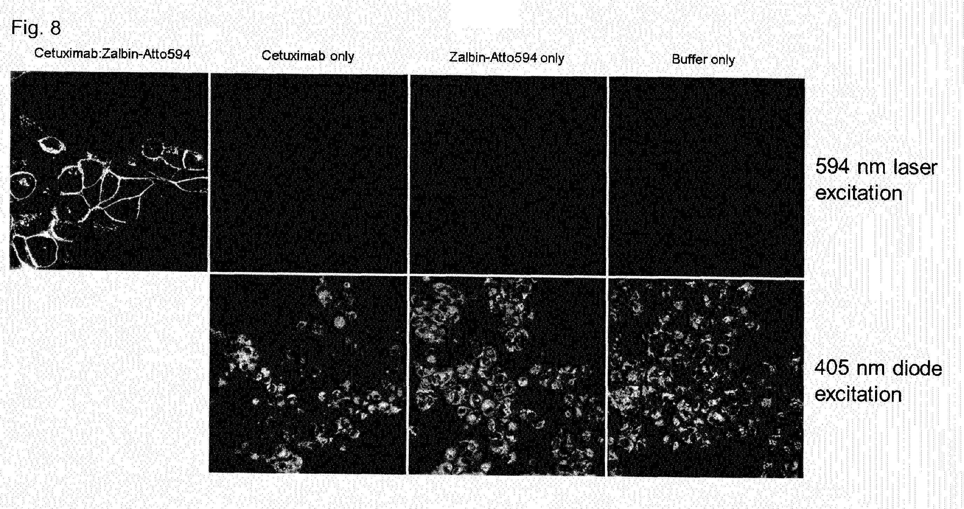

As MD simulations predict both domains not to interfere with each other in a way that domain functions are impeded, the capability of binding of Z-domain to Fc fragments of antibodies was tested *in vitro* using fluorescence spectroscopy. For this purpose Zalbin was labelled with Atto-465 at its N-terminus and titrated with the monoclonal anti EGFR-IgG-antibody Cetuximab as a model compound. Changes in fluorescence anisotropy were followed for each titration step (figure 7). Nonlinear regression using a hyperbolic binding curve revealed a dissociation constant of 470 nM for the complex of Cetuximab and Atto-465-Zalbin. To verify the ability of Cetuximab:Zalbin to target potential cancer cells, binding of this complex to EGFR-expressing cells was demonstrated. The binding event of the complex to A431 cancer cells was visualized by confocal microscopy. Therefore, a D72C mutant of Zalbin was labelled with Atto-594 and preincubated with Cetuximab prior to incubation of the D72C Cetuximab:Zalbin complex to the cancer cells. After washing off cells fluorescence of Atto-594 was detected (figure 8). Staining is almost exclusively observed at the cellular membrane as confirmed by z-stack calculation/recording. Portions of fluorescence observed inside the cells result from binding events to EGF receptors partially internalised by the cells over time [24]. Thus, fluorescence anisotropy binding studies and fluorescence microscopy experiments provide a proof of principle for the modular probes concept employing the Z-domain of *staphylococcus aureus* as a mediator for various targeting applications.

Integrity of the parvalbumin-domain function

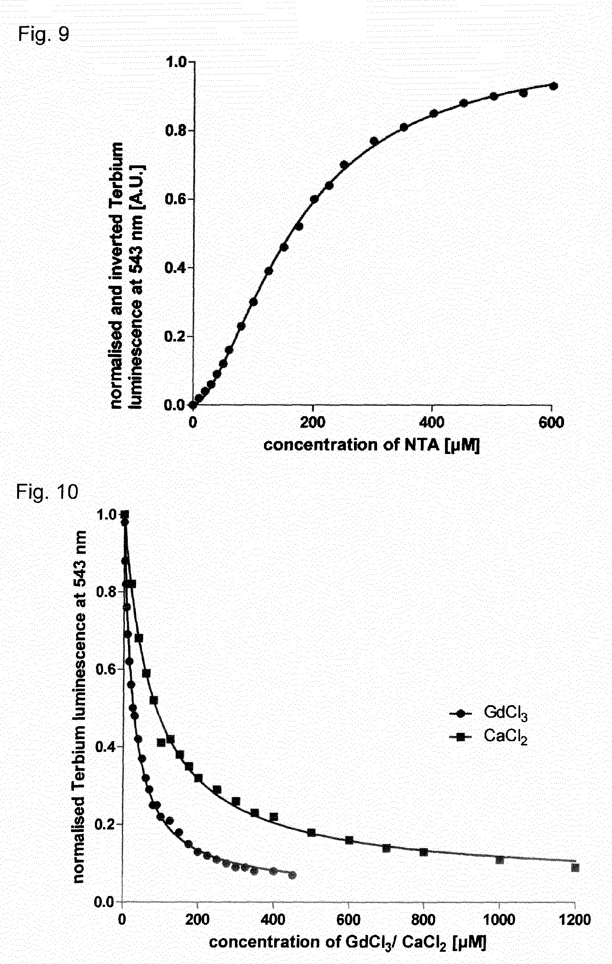

Due to the proposed application of the fusion construct as a $T_1$ contrast agent the capability of the of S55D/E59D alpha-parvalbumin domain to bind paramagnetic ions, especially trivalent $Gd^{3+}$, with high affinity should was tested. As Terbium (III) has a very similar ion radius when compared to Gadolinium (III) and shows detectable luminescence upon binding to a chelating compound, the affinity for complex formation of Zalbin and $Tb^{3+}$ was determined first. Assuming a very high affinity for this interaction, the affinity of $Tb^{3+}$ was measured in a competition assay using nitrilotriacetic acid as a competitor for the Zalbin. Figure 9 shows the titration of 3 $\mu$M $Tb^{3+}$ and 8 $\mu$M Zalbin with NTA. The curve was normalised and inverted prior to fitting. Alpha-parvalbumin of Zalbin binds metal ions cooperatively [25] and in agreement with wild-type data from literature [25] the measured affinities of the EF- and CD-site were found indistinguishable in the binding assay. An average affinity of both the EF- and mutated high affinity CD-site for $Tb^{3+}$ was calculated by fitting the titration curve with a Hill equation. Using the fitted apparent affinity of NTA:$Tb^{3+}$ and the binding affinity of 5.6 $\times$ $10^{-12}$ M given in the literature for this complex [14], the binding affinity of Zalbin:$(Tb^{3+})_2$ was calculated to be about 2.63 $\times$ $10^{-13}$ M.

The affinity of $Gd^{3+}$ and for comparison of $Ca^{2+}$ were measured using a competition assay where 4 $\mu$M Zalbin and 10 $\mu$M $Tb^{3+}$ were titrated with $Gd^{3+}$ or $Ca^{2+}$ respectively (figure 10). Data were fitted using a hyperbolic equation for competition titrations yielding values of 6.50 $\times$ $10^{-13}$ M for Zalbin:$(Gd^{3+})_2$ and 2.16 $\times$ $10^{-13}$ M for Zalbin:$(Ca^{2+})_2$. The measured affinity constants confirm that high affine binding of metal ions is conserved in the two-domain fusion construct.

Relaxometry

As Zalbin was designed to function as a targeting $T_1$ contrast agent it should feature a high relaxivity $r_1$. The higher this value the better the contrast that is generated by the agent. The relaxometric properties of Zalbin were investigated *in vitro* using MRI at field strengths of 1.5 T, 3 T as well as 7 T. A diluted solution with rising amounts of Zalbin:$(Gd^{3+})_2$ in each row on a 4 $\times$ 6 well plate was applied to an inversion recovery turbo spin echo experiment. Figure 11 shows the influence of Zalbin:$(Gd^{3+})_2$ on $T_1$. The inversion times (TIs) chosen for this figure for 1,5 T, 3 T and 7 T were 1900 ms, 1800 ms and 1950 ms, respectively. The TIs are selected for zeroing the MRI signal of the well containing buffer exclusively whereby it appears black. Increasing brightness observed in the wells along each row displays increasing contrast as a function of the Zalbin:$(Gd^{3+})_2$ concentration. While at 1.5 T the limit for detecting observable contrast is found at a protein concentration below 0.5 $\mu$M, this concentration rises to a value of 0.5 to 1 $\mu$M at 3 T and reaches values between 1 to 5 $\mu$M at a field strength of 7 T. Longitudinal relaxivities $r_1$ of Zalbin:$(Gd^{3+})2$ yielded values of 50.63 $\pm$ 1.25 $s^1mM^1$ for 1.5 T, 24.87 $\pm$ 0.49 $s^1mM^1$ for 3 T and 8.8 $\pm$ 1.48 $s^1mM^1$ for 7 T, respectively.

Especially at 1.5 and 3 T relaxivities of Zalbin are significantly higher than those of conventionally used small molecular contrast agents like DTPA-$Gd^{3+}$ (Magnevist) and DOTA-$Gd^{3+}$ (Dotarem). Relaxivities of these contrast agents are below 10 $s^{-1}mM^{-1}$ [26]. From $r_1$ values it can be estimated whether the limiting concentrations of Zalbin:$(Gd^{3+})_2$ retrieved from figure 11 are sufficient to produce observable contrast when bound to A431 cancer cells. This cell line expresses about 1.6 - 2.6 $\times$ $10^6$ EGF receptors per cell [27], [28]). Assuming a cell diameter of 15 - 25 microns, the concentration of EGF receptors averaged over the volume of a cell is between 0.32 and 2.44 $\mu$M. According to this simple model metastases could principally receive higher contrast towards normal tissue at 1.5 or 3 T when using Zalbin $(Gd^{3+})_2$ in combination with Cetuximab as a contrast agent when compared to the application of commercial available small molecular weight contrast agents. Detection of metastases would then be limited by the resolution of the MRI scanner, which is in the sub-millimetre range for the three field strengths mentioned. Metastases that are large enough to be displayed in the respective MR images, are sufficient to produce a detectable contrast in normal tissues at Zalbin $(Gd^{3+})_2$ protein concentrations of 0.32 - 2.44 $\mu$M inside the metastase.

Stability of Zalbin

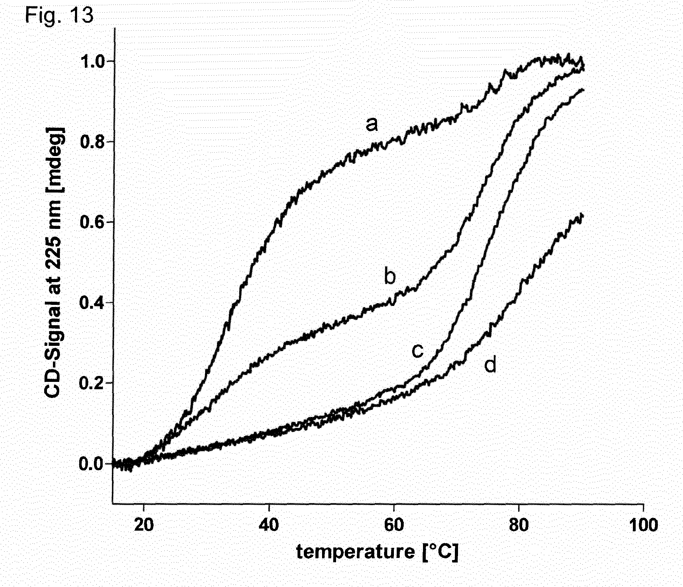

With regard to a possible *in vivo* application the stability of Zalbin towards temperature and serum conditions in FCS was investigated. Zalbin at a concentration of 2 mg/ml was incubated in 50% FCS and 37 °C over a time period of 24 h. After certain times aliquots were taken and tested on degradation of the Zalbin band on a Schägger-Jagov gel (figure 12). Even after 24 h, allowing enough time for MRT examination and subsequent excretion of the contrast agent, there is no visible degradation of the fusion protein. Thermal stability of Zalbin was measured employing CD spectroscopy. The CD signal at 225 nm was recorded during heating of the sample. Figure 13 shows an overlay of the melting curves of Zalbin, the Z-domain, the S55D/E59D mutant of alpha-parvalbumin and Zalbin:$(Gd^{3+})_2$. The Z-domain exhibits a melting temperature of about 73 °C. The melting points of S55D/E59D alpha-parvalbumin and its complement in the fusion protein Zalbin are at 27 °C. Although the apo-form of the latter is not stable at body temperature, binding of a metal ion like $Gd^{3+}$ to the EF- and CD-site stabilizes the holo-form of the domain, which is the relevant form for an *in vivo* application. The melting points of Zalbin:$(Gd^{3+})_2$ ascends beyond 75 °C. Zalbin:$(Gd^{3+})_2$ refolds completely reversible after samples are cooled down to body temperature.

Kinetic stability as important predictor for *in vivo* stability of the Zalbin:$(Gd^{3+})_2$ complex was investigated [29]. Again, Terbium (III) was chosen, as its dissociation can easily be monitored by measuring luminescence. In FCS half-lives of about 2.5 - 3 Min were determined for the protein-metal complex. The low half-life is caused by the presence of $Ca^{2+}$ and metal ion binding proteins in the serum. To explore which of these both components is mainly responsible for pulling out of $Tb^{3+}$, serum proteins were separated from the liquid part by centrifugation of FCS through a 3 kDa MWCO centricon membrane. Subsequently dissociation of the Zalbin:$(Tb^{3+})_2$ complex was measured in the flow-through as well as in a Tris buffered solution containing the washed serum proteins. In the flow-through the same half-life of Zalbin:$(Tb^{3+})_2$ was observed as measured in presence of complete FCS, whereas in the presence of serum proteins the metal ion half-life of Zalbin:$(Tb^{3+})_2$ is about 90 minutes. Therefore, Zalbin:$(Gd^{3+})_2$ requires optimisation concerning the kinetic stability of the complex.

General Findings and Consequences

The designed two-domain protein Zalbin is folded correctly and both domains are functional. Its apparently higher molecular weight in gelfiltration can be attributed to the fact that the two domains are connected by a flexible polyglycine linker. This causes a larger hydrodynamic radius of the complete protein. A monomeric form of the protein is of advantage concerning the amount of protein required to form the stoichiometry of two Zalbin proteins per IgG antibody. Multimerisation may require more protein or more time to saturate all antibody binding sites and thus would increase the costs for a possible application. Another advantage of the monomeric form is that Zalbin can be excreted from the system by renal filtration without the need of enzymatic degradation in the blood.

Further, Zalbin is stable towards serum conditions and in its holo-form Zalbin:$(Gd^{3+})_2$ it exhibits a melting temperature beyond 75 °C. Thus, the protein is stable long enough for an MRT examination and subsequent renal excretion.

In contrast to low molecular weight chelates, the use of S55D/E59D mutant of rat alpha-parvalbumin allowed addressing high relaxivity at clinically relevant field strengths (1.5 and 3 Tesla) because the rotational correlation time of Gadolinium (III) was lowered.

High longitudinal relaxivities $r_1$ at 1.5 and 3 T combined with its IgG binding feature proved Zalbin to be a possible tumour targeting $T_1$ contrast agent for detection of metastases in MRI. Immune reactivity towards the bacterial Z-domain is not considered a problem due to the short time of the application and because the protein is probably applied only once. Nevertheless, in a next step elimination of HLA epitopes of Zalbin will be addressed using bioinformatical methods. A further task to improve Zalbin for in vivo applications is to optimise the kinetic stability of the Zalbin:$(Gd^{3+})_2$ complex by site directed mutagenesis to cage the metal ion inside the binding sites.

Zalbin fulfils the task of a selective protein targeting and high relaxivity contrast agent for molecular imaging methods, in particular for MRI, very well.

**References**

1 Nilsson, B., Moks, T., Jansson, B., Abrahmsen, L., Elmblad, A., Holmgren, E., Henrichson, C., Jones, T. A. and Uhlen, M. (1987 Feb-Mar) A synthetic IgG-binding domain based on staphylococcal protein A. Protein Engineering 1, 107-113

2 Caravan, P., Farrar, C. T., Frullano, L. and Uppal, R. (2009) Influence of molecular parameters and increasing magnetic field strength on relaxivity of gadolinium- and manganese-based T-1 contrast agents. CONTRAST MEDIA MOL 14, 89-100

3 Lee, Y. H., Tanner, J. J., Larson, J. D. and Henzl, M. T. (2004) Crystal structure of a high-affinity variant of rat alpha-parvalbumin. BIOCHEMISTRY-US 43, 10008-10017

4 BERNSTEIN, F. C., KOETZLE, T. F., WILLIAMS, G. J., MEYER, E. F., BRICE, M. D., RODGERS, JR, KENNARD, O., SHIMANOUCHI, T. and TASUMI, M. (1977) PROTEIN DATA BANK - COMPUTER-BASED ARCHIVAL FILE FOR MACROMOLECULAR STRUCTURES. EUR J BIOCHEM 80, 319-324

5 Marti-Renom, M. A., Stuart, A. C., Fiser, A., Sanchez, R., Melo, F. and Sali, A. (2000) Comparative protein structure modeling of genes and genomes. ANNU REV BIOPH BIOM 29, 291-325

6 Zheng, D. Y., Aramini, J. M. and Montelione, G. T. (2004) Validation of helical tilt angles in the solution NMR structure of the Z domain of Staphylococcal protein A by combined analysis of residual dipolar coupling and NOE data. PROTEIN SCI 13, 549-554

7 KABSCH, W. and SANDER, C. (1983) DICTIONARY OF PROTEIN SECONDARY STRUCTURE - PATTERN-RECOGNITION OF HYDROGEN-BONDED AND GEOMETRICAL FEATURES. BIOPOLYMERS 22, 2577-2637

8 Daura, X., Gademann, K., Jaun, B., Seebach, D., van Gunsteren, W. F. and Mark, A. E. (1999) Peptide folding: When simulation meets experiment. ANGEW CHEM INT EDIT 38, 236-240

9 Lindahl, E., Hess, B. and van der Spoel, D. (2001) GROMACS 3.0: a package for molecular simulation and trajectory analysis. J MOL MODEL 7, 306-317

10 Daura, X., Mark, A. E. and van Gunsteren, W. F. (1998) Parametrization of aliphatic CHn united atoms of GROMOS96 force field. J COMPUT CHEM 19, 535-547

11 RYCKAERT, J. P., CICCOTTI, G. and BERENDSEN, H. J. (1977) NUMERICAL-INTEGRATION OF CARTESIAN EQUATIONS OF MOTION OF A SYSTEM WITH CONSTRAINTS - MOLECULAR-DYNAMICS OF N-ALKANES. J COMPUT PHYS 23, 327-341

12 Plazinskia, W. and Drachb, M. (2012) Thermodynamic aspects of calcium binding by poly($\alpha$-L-guluronate) chains. A molecular simulation study. Applied Surface Science.

13 Fasman, G. D. (1976) Handbook of biochemistry and molecular biology. Proteins, Volume I, CRC Press, Cleveland, Ohio

14 Martell, A. E., Smith, R. M. and Motekaitis, R. J. (1993) NIST Standard Reference Data, NIST Standard Reference Database 46: NIST Critically Selected Stability Constants of Metal Complexes Version 8.0

15 Yung-Chi C, P. W. H. (1973) Relationship between the inhibition constant (KI) and the concentration of inhibitor which causes 50 per cent inhibition (I50) of an enzymatic reaction. Biochemical Pharmacology, 3099-3108

16 Basu, G., Allen, M., Willits, D., Young, M. and Douglas, T. (2003) Metal binding to cowpea chlorotic mottle virus using terbium(III) fluorescence. J BIOL INORG CHEM 8, 721-725

17 Bernstein, M. A., King, K. F. and Zhou, X. J. (2004) Handbook of MRI pulse sequences, chapter 14.2.1, page 608-611. Academic Press, Amsterdam ; Boston

18 DEISENHOFER, J. (1981) CRYSTALLOGRAPHIC REFINEMENT AND ATOMIC MODELS OF A HUMAN FC FRAGMENT AND ITS COMPLEX WITH FRAGMENT-B OF PROTEIN-A FROM STAPHYLOCOCCUS-AUREUS AT 2.9-A AND 2.8-A RESOLUTION. BIOCHEMISTRY-US 20, 2361-2370

19 Ragab, Y., Emad, Y. and Banakhar, A. (2007) Inflammatory synovitis due to underlying lipoma arborescens (gadolinium-enhanced MRI features): report of two cases. CLIN RHEUMATOL 26, 1791-1794

20 Brücher, E. (2002) Kinetic Stabilities of Gadolinium (III) Chelates Used as MRI Contrast Agents. Topics in Current Chemistry, 103-122

21 MICSKEI, K., HELM, L., BRUCHER, E. and MERBACH, A. E. (1993) O-17 NMR-STUDY OF WATER EXCHANGE ON [GD(DTPA)(H2O)]2- AND [GD(DOTA)(H2O)]- RELATED TO NMR IMAGING. INORG CHEM 32, 3844-3850

22 Helm, L. (2010) Optimization of gadolinium-based MRI contrast agents for high magnetic-field applications. FUTURE MED CHEM 2, 385-396

23 HORROCKS, W. D. and SUDNICK (1979) LANTHANIDE ION PROBES OF STRUCTURE IN BIOLOGY - LASER-INDUCED LUMINESCENCE DECAY CONSTANTS PROVIDE A DIRECT MEASURE OF THE NUMBER OF METAL-COORDINATED WATER-MOLECULES. J AM CHEM SOC 101, 334-340

24 MASUI, H., CASTRO, L. and MENDELSOHN, J. (1993) CONSUMPTION OF EGF BY A431 CELLS - EVIDENCE FOR RECEPTOR RECYCLING. J CELL BIOL 120, 85-93

25 Henzl, M. T., Agah, S. and Larson, J. D. (2003) Characterization of the metal ion-binding domains from rat alpha- and beta-parvalbumins. BIOCHEMISTRY-US 42, 3594-3607

26 Laurent, S., Elst, L. V. and Muller, R. N. (2006) Comparative study of the physicochemical properties of six clinical low molecular weight gadolinium contrast agents. CONTRAST MEDIA MOL I1; 128-137

27 KWOK, T. T. and SUTHERLAND, R. M. (1991) DIFFERENCES IN EGF RELATED RADIOSENSITIZATION OF HUMAN SQUAMOUS CARCINOMA-CELLS WITH HIGH AND LOW NUMBERS OF EGF RECEPTORS. BRIT J CANCER 64, 251-254

28 Haigler, H., Ash, J. F., Singer, S. J. and Cohen, S. (1978) Visualization by fluorescence of the binding and internalization of epidermal growth factor in human carcinoma cells A-431. Proc Natl Acad Sci USA 75, 3317-3321

29 Sherry, A. D., Caravan, P. and Lenkinski, R. E. (2009) Primer on Gadolinium Chemistry. J MAGN RESON IMAGING 30, 1240-1248

30 Liepold, L.O., Abedin, M.J., Buckhouse, E.D., Frank, J.A., Young, M.J., Douglas T. Supramolecular protein cage composite MR contrast agents with extremely efficient relaxivity properties. Nano Lett. 9(12):4520-4526.

## Claims

1.  A polypeptide comprising:

    (a) at least one first domain for binding to at least one contrast agent; and

(b) at least one second domain for binding to at least one target structure-binding compound.

2. The polypeptide according to claim 1, wherein the contrast agent is a metal atom or metal ion, preferably wherein the metal atom or metal ion is a rare earth atom or rare earth ion or $Fe^{2+}$, $Fe^{3+}$ or $Mn^{2+}$, more preferably $Gd^{3+}$, $Dy^{3+}$, $Yb^{3+}$ or $Ho^{3+}$, in particular $Gd^{3+}$.

3. The polypeptide according to claim 1 or 2, wherein the first domain comprises parvalbumin, a calcium-binding polypeptide of the EF hand family, a calcium-binding annexin polypeptide or a calcium-binding plasma membrane ATPase or a contrast agent-binding fraction or mutant of any thereof, in particular wherein the first domain comprises alpha parvalbumin or a contrast agent-binding fraction or mutant thereof.

4. The polypeptide according to at least one of claims 1 to 3, wherein the target structure-binding compound is an antibody or a fragment or mutant thereof binding to an antigen, in particular wherein said target structure-binding compound comprises an Fc fragment or mutant thereof that is binding to the second domain.

5. The polypeptide according to claim 4, wherein said second domain comprises an Fc fragment-binding polypeptide domain of protein A of *Staphylococcus aureus.*

6. A probe for molecular imaging comprising the following components:

   (a) at least one polypeptide according to any one of claims 1 to 5; and
   (b) at least one contrast agent binding to a first domain of said polypeptide,
   and optionally further comprising:
   (c) at least one target structure-binding compound binding to a second domain of said polypeptide.

7. The probe according to claim 6, wherein molecular imaging is selected from the group consisting of:

   magnetic resonance imaging; and
   radiography, in particular computer tomography.

8. A nucleic acid molecule encoding for a polypeptide according to any one of claims 1 to 5.

9. A vector comprising the nucleic acid molecule according to claim 8.

10. A host cell comprising the vector according to claim 9.

11. A diagnostic composition comprising:

    (a) at least one polypeptide according to any one of claims 1 to 5, at least one probe according to claim 6 or 7, at least one nucleic acid molecule according to claim 8, at least one vector according to claim 9 and/or at least one host cell according to claim 10;
    (b) at least one contrast agent binding to a first domain of the polypeptide according to any one of claims 1 to 5; and
    (c) a pharmaceutically acceptable carrier.

12. The diagnostic composition according to claim 11, further comprising at least one component selected from the group consisting of:

    (d) at least one target structure-binding compound;
    (e) at least one nucleic acid molecule encoding for the target structure-binding compound;
    (f) at least one vector comprising the nucleic acid molecule encoding for the target structure-binding compound; and
    (g) at least one host cell suitable for expressing the target structure-binding compound.

13. The polypeptide according to any one of claims 1 to 5, the probe according to claim 6 or 7, the nucleic acid according to claim 8, the vector according to claim 9, the host cell according to claim 10 and/or the diagnostic composition according to claim 11 or 12 for use in a method for the diagnosis of a patient suffering from or being at risk of developing at least one of the physiological or pathological condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration.

14. A method for producing the polypeptide according to any one of claims 1 to 5, said method comprising the following steps:

(i) providing a host cell according to claim 10,
(ii) culturing said host cell under conditions that allow said host cell to express the polypeptide according to any one of claims 1 to 5; and
(iii) recovering said polypeptide from said culture.

15. Use of a polypeptide according to any one of claims 1 to 5, a probe according to claim 6 or 7, a nucleic acid molecule according to claim 8, a vector according to claim 9, a host cell according to claim 10 and/or a diagnostic composition according to claim 11 or 12 for detecting the localization and/or the local concentration of a molecular target structure *in vitro,*
preferably wherein a local alteration in the concentration compared to a standard indicates a specific physiological or pathological condition,
in particular wherein said specific physiological or pathological condition is a condition selected from the group consisting of cancer, formation of metastases, neoplasia, Parkinson disease, Alzheimer disease, arteriosclerosis, bone growth, tooth growth, cartilage degeneration, rheumatoid arthritis, inflammatory lesions, multiple sclerosis and one or more other conditions associated with a local alteration of the molecular target structure concentration.

Fig. 1

Parvalbumin    Ca(2+)    Z-Domain

Polyglycine Linker

Fig. 2

Fig. 3

Fig. 4

Fig. 5

Fig. 6

Fig. 7

Fig. 8

Fig. 9

Fig. 10

Fig. 11

1.5 T    3 T    7 T

Fig. 12

Fig. 13

## EUROPEAN SEARCH REPORT

**Application Number**

EP 12 00 5859

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/146099 A2 (UNIV GEORGIA STATE RES FOUND [US]; YANG JENNY JIE [US]; LIU ZHIREN [US] 3 December 2009 (2009-12-03) * whole doc, in particular claim 4, example 4.2, p. 32-33, 88-92 * | 1-15 | INV. A61K49/00 |
| X | WO 2007/009058 A2 (UNIV GEORGIA STATE RES FOUND [US]; YANG JENNY J [US]; LIU ZHI-REN [US]) 18 January 2007 (2007-01-18) | 1 | |
| A | * whole do, in particular p. 13, lines 6-10 and claims * | 2-15 | |
| X | JENNY J YANG ET AL: "Rational design of protein-based MRI contrast agents", JOURNAL OF THE AMERICAN CHEMICAL SOCIETY, ACS PUBLICATIONS, US, vol. 130, no. 29, 23 July 2008 (2008-07-23), pages 9260-9267, XP002627489, ISSN: 0002-7863, DOI: 10.1021/JA800736H [retrieved on 2008-06-25] | 1 | |
| A | * whole doc, in particular abstract and fig. 1c * | 2-15 | TECHNICAL FIELDS SEARCHED (IPC)  A61K |
| T | DANIEL GRUM ET AL: "Design of a Modular Protein-Based MRI Contrast Agent for Targeted Application", PLOS ONE, vol. 8, no. 6, 6 June 2013 (2013-06-06), page e65346, XP055084831, ISSN: 1932-6203, DOI: 10.1371/journal.pone.0065346 * the whole document * | 1-15 | |

The present search report has been drawn up for all claims

| Place of search | Date of completion of the search | Examiner |
|---|---|---|
| Munich | 23 October 2013 | Lüdemann, Susanna |

EPO FORM 1503 03.82 (P04C01)

**ANNEX TO THE EUROPEAN SEARCH REPORT**
**ON EUROPEAN PATENT APPLICATION NO.**

EP 12 00 5859

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2013

| Patent document cited in search report | | Publication date | Patent family member(s) | | Publication date |
|---|---|---|---|---|---|
| WO 2009146099 | A2 | 03-12-2009 | EP | 2257316 A2 | 08-12-2010 |
| | | | US | 2011097742 A1 | 28-04-2011 |
| | | | WO | 2009146099 A2 | 03-12-2009 |
| WO 2007009058 | A2 | 18-01-2007 | CA | 2614486 A1 | 18-01-2007 |
| | | | CN | 101222878 A | 16-07-2008 |
| | | | EP | 1901659 A2 | 26-03-2008 |
| | | | US | 2009087382 A1 | 02-04-2009 |
| | | | US | 2012269735 A1 | 25-10-2012 |
| | | | WO | 2007009058 A2 | 18-01-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIEPOLD et al.** *Nano Lett,* 2009, vol. 9 (12), 4520-6
- **NILSSON, B. ; MOKS, T. ; JANSSON, B. ; AB-RAHMSEN, L. ; ELMBLAD, A. ; HOLMGREN, E. ; HENRICHSON, C. ; JONES, T. A. ; UHLEN, M.** A synthetic IgG-binding domain based on staphylococcal protein A. *Protein Engineering,* February 1987, vol. 1, 107-113
- **CARAVAN, P. ; FARRAR, C. T. ; FRULLANO, L. ; UPPAL, R.** Influence of molecular parameters and increasing magnetic field strength on relaxivity of gadolinium- and manganese-based T-1 contrast agents. *CONTRAST MEDIA MOL,* 2009, vol. 14, 89-100
- **LEE, Y. H. ; TANNER, J. J. ; LARSON, J. D. ; HEN-ZL, M. T.** Crystal structure of a high-affinity variant of rat alpha-parvalbumin. *BIOCHEMISTRY-US,* 2004, vol. 43, 10008-10017
- **BERNSTEIN, F. C. ; KOETZLE, T. F. ; WILLIAMS, G. J. ; MEYER, E. F. ; BRICE, M. D. ; RODGERS, JR ; KENNARD, O. ; SHIMANOUCHI, T. ; TASUMI, M.** PROTEIN DATA BANK - COMPUTER-BASED ARCHIVAL FILE FOR MACROMOLECULAR STRUCTURES. *EUR J BIOCHEM,* 1977, vol. 80, 319-324
- **MARTI-RENOM, M. A. ; STUART, A. C. ; FISER, A. ; SANCHEZ, R. ; MELO, F. ; SALI, A.** Comparative protein structure modeling of genes and genomes. *ANNU REV BIOPH BIOM,* 2000, vol. 29, 291-325
- **ZHENG, D. Y. ; ARAMINI, J. M. ; MONTELIONE, G. T.** Validation of helical tilt angles in the solution NMR structure of the Z domain of Staphylococcal protein A by combined analysis of residual dipolar coupling and NOE data. *PROTEIN SCI,* 2004, vol. 13, 549-554
- DICTIONARY OF PROTEIN SECONDARY STRUC-TURE - PATTERN-RECOGNITION OF HYDRO-GEN-BONDED AND GEOMETRICAL FEATURES. **KABSCH, W. ; SANDER, C.** BIOPOLYMERS. 1983, vol. 22, 2577-2637
- **DAURA, X. ; GADEMANN, K. ; JAUN, B. ; SEE-BACH, D. ; VAN GUNSTEREN, W. F. ; MARK, A. E.** Peptide folding: When simulation meets experiment. *ANGEW CHEM INT EDIT,* 1999, vol. 38, 236-240

- **LINDAHL, E. ; HESS, B. ; VAN DER SPOEL, D.** GROMACS 3.0: a package for molecular simulation and trajectory analysis. *J MOL MODEL,* 2001, vol. 7, 306-317
- **DAURA, X. ; MARK, A. E. ; VAN GUNSTEREN, W. F.** Parametrization of aliphatic CHn united atoms of GROMOS96 force field. *J COMPUT CHEM,* 1998, vol. 19, 535-547
- **RYCKAERT, J. P. ; CICCOTTI, G. ; BERENDSEN, H. J.** NUMERICAL-INTEGRATION OF CARTESIAN EQUATIONS OF MOTION OF A SYSTEM WITH CONSTRAINTS - MOLECULAR-DYNAMICS OF N-ALKANES. *J COMPUT PHYS,* 1977, vol. 23, 327-341
- **PLAZINSKIA, W. ; DRACHB, M.** Thermodynamic aspects of calcium binding by poly($\alpha$-L-guluronate) chains. A molecular simulation study. *Applied Surface Science,* 2012
- **FASMAN, G. D.** Handbook of biochemistry and molecular biology. Proteins. CRC Press, 1976, vol. I
- **MARTELL, A. E. ; SMITH, R. M. ; MOTEKAITIS, R. J.** *NIST Standard Reference Data, NIST Standard Reference Database 46: NIST Critically Selected Stability Constants of Metal Complexes,* 1993
- **YUNG-CHI C, P. W. H.** Relationship between the inhibition constant (KI) and the concentration of inhibitor which causes 50 per cent inhibition (I50) of an enzymatic reaction. *Biochemical Pharmacology,* 1973, 3099-3108
- **BASU, G. ; ALLEN, M. ; WILLITS, D. ; YOUNG, M. ; DOUGLAS, T.** Metal binding to cowpea chlorotic mottle virus using terbium(III) fluorescence. *J BIOL IN-ORG CHEM,* 2003, vol. 8, 721-725
- **BERNSTEIN, M. A. ; KING, K. F. ; ZHOU, X. J.** Handbook of MRI pulse sequences. Academic Press, 2004, 608-611
- **DEISENHOFER, J.** CRYSTALLOGRAPHIC RE-FINEMENT AND ATOMIC MODELS OF A HUMAN FC FRAGMENT AND ITS COMPLEX WITH FRAGMENT-B OF PROTEIN-A FROM STAPHYLOCOCCUS-AUREUS AT 2.9-A AND 2.8-A RESOLUTION. *BIOCHEMISTRY-US,* 1981, vol. 20, 2361-2370
- **RAGAB, Y. ; EMAD, Y. ; BANAKHAR, A.** Inflammatory synovitis due to underlying lipoma arborescens (gadolinium-enhanced MRI features): report of two cases. *CLIN RHEUMATOL,* 2007, vol. 26, 1791-1794

- **BRÜCHER, E.** Kinetic Stabilities of Gadolinium (III) Chelates Used as MRI Contrast Agents. *Topics in Current Chemistry,* 2002, 103-122
- **MICSKEI, K. ; HELM, L. ; BRUCHER, E. ; MERBACH, A. E.** O-17 NMR-STUDY OF WATER EXCHANGE ON [GD(DTPA)(H2O)]2- AND [GD(DOTA)(H2O)]- RELATED TO NMR IMAGING. *INORG CHEM,* 1993, vol. 32, 3844-3850
- **HELM, L.** Optimization of gadolinium-based MRI contrast agents for high magnetic-field applications. *FUTURE MED CHEM,* 2010, vol. 2, 385-396
- **HORROCKS, W. D. ; SUDNICK.** LANTHANIDE ION PROBES OF STRUCTURE IN BIOLOGY - LASER-INDUCED LUMINESCENCE DECAY CONSTANTS PROVIDE A DIRECT MEASURE OF THE NUMBER OF METAL-COORDINATED WATER-MOLECULES. *J AM CHEM SOC,* 1979, vol. 101, 334-340
- **MASUI, H. ; CASTRO, L. ; MENDELSOHN, J.** CONSUMPTION OF EGF BY A431 CELLS - EVIDENCE FOR RECEPTOR RECYCLING. *J CELL BIOL,* 1993, vol. 120, 85-93
- **HENZL, M. T. ; AGAH, S. ; LARSON, J. D.** Characterization of the metal ion-binding domains from rat alpha- and beta-parvalbumins. *BIOCHEMISTRY-US,* 2003, vol. 42, 3594-3607
- **LAURENT, S. ; ELST, L. V. ; MULLER, R. N.** Comparative study of the physicochemical properties of six clinical low molecular weight gadolinium contrast agents. *CONTRAST MEDIA MOL I1,* 2006, 128-137
- **KWOK, T. T. ; SUTHERLAND, R. M.** DIFFERENCES IN EGF RELATED RADIOSENSITIZATION OF HUMAN SQUAMOUS CARCINOMA-CELLS WITH HIGH AND LOW NUMBERS OF EGF RECEPTORS. *BRIT J CANCER,* 1991, vol. 64, 251-254
- **HAIGLER, H. ; ASH, J. F. ; SINGER, S. J. ; COHEN, S.** Visualization by fluorescence of the binding and internalization of epidermal growth factor in human carcinoma cells A-431. *Proc Natl Acad Sci USA,* 1978, vol. 75, 3317-3321
- **SHERRY, A. D. ; CARAVAN, P. ; LENKINSKI, R. E.** Primer on Gadolinium Chemistry. *J MAGN RESON IMAGING,* 2009, vol. 30, 1240-1248
- **LIEPOLD, L.O. ; ABEDIN, M.J. ; BUCKHOUSE, E.D. ; FRANK, J.A. ; YOUNG, M.J. ; DOUGLAS T.** Supramolecular protein cage composite MR contrast agents with extremely efficient relaxivity properties. *Nano Lett.,* vol. 9 (12), 4520-4526